(19) Europäisches Patentamt / European Patent Office / Office européen des brevets

(11) **EP 1 688 499 B1**

(12) # EUROPEAN PATENT SPECIFICATION

(45) Date of publication and mention of the grant of the patent:
**29.12.2010 Bulletin 2010/52**

(21) Application number: **06003507.8**

(22) Date of filing: **11.09.1997**

(51) Int Cl.:
*C12N 15/53* (2006.01) *C12N 9/04* (2006.01)
*C12N 1/21* (2006.01) *C12N 5/10* (2006.01)
*C12P 7/24* (2006.01) *C12P 7/26* (2006.01)
*C12P 7/40* (2006.01) *C12P 17/00* (2006.01)

(54) **Alcohol-aldehyd-dehydrogenases**

Alkohol-Aldehyd-Deshydrogenasen

Déshydrogénase d'alcool et d'aldéhyde

(84) Designated Contracting States:
**AT BE CH DE DK ES FR GB IT LI NL**

(30) Priority: **19.09.1996 EP 96115001**

(43) Date of publication of application:
**09.08.2006 Bulletin 2006/32**

(62) Document number(s) of the earlier application(s) in accordance with Art. 76 EPC:
**97115801.9 / 0 832 974**

(73) Proprietor: **DSM IP Assets B.V.**
**6411 TE Heerlen (NL)**

(72) Inventors:
- **Asakura, Akira**
**251-0033 Kanagawa-ken (JP)**
- **Hoshino, Tatsuo**
**248-0027 Kamakura-shi,**
**Kanagawa-prefect. (JP)**
- **Ojima, Setsuko**
**Fujisawa-shi,**
**Kanagawa-ken (JP)**
- **Shinjoh, Masako**
**Kamakura,**
**247-0061 Kanagawa (JP)**
- **Tomiyama, Noribumi**
**251-0015 Fujisawa-shi,**
**Kanagawa-ken (JP)**

(74) Representative: **Schwander, Kuno et al**
**DSM Nutritional Products Ltd.,**
**Wurmisweg 576**
**4303 Kaiseraugst (CH)**

(56) References cited:
**EP-A- 0 448 969 EP-A- 0 606 621**

- **STOORVOGEL J. ET AL.: "Characterization of the gene encoding quinohaemoprotein ethanol dehydrogenase of Comamonas testosteroni" EUR. J. BIOCHEM., vol. 235, 1996, page 690-698, XP002110757**
- **"Alcohol dehydrogenase complex structural gene-used in plasmid and enhancing efficiency of acetic acid fermentation for transformed acetic acid bacteria" GENESEQ DATABASE, 16 April 1992 (1992-04-16), XP002110758**
- **TAMAKI T. ET AL.: "Cloning and sequencing of the gene cluster encoding two subunits of membrane-bound alcohol dehydrogenase from Acetobacter polyoxogenes" BIOCHIM. BIOPHYS. ACTA, vol. 1088, 1991, pages 292-300, XP002110759**
- **KONDO K. ET AL: "CHARACTERIZATION OF THE GENES ENCODING THE THREE-COMPONENT MEMBRANE-BOUND ALCOHOL DEHYDROGENASE FROM GLUCONOBACTER SUBOXYDANS AND THEIR EXPRESSION IN ACETOBACTER PASTEURIANUS" APPLIED AND ENVIRONMENTAL MICROBIOLOGY, vol. 63, no. 3, March 1997 (1997-03), pages 1131-1138, XP002914612**
- **REID M.F.: "Molecular Characterization of Microbial Alcohol Dehydrogenases" CRIT. REV. MICROBIOL., vol. 20, no. 1, 1994, page 13-56, XP002110760**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 688 499 B1

SEQUENCE LISTING

(1) GENERAL INFORMATION

(i) APPLICANT

(A) NAME: DSM IP Assets B.V.
(B) STREET: Het Overloon 1
(C) CITY: TE Heerlen
(E) COUNTRY: The Netherlands
(F) POSTAL CODE: NL-6411

(ii) TITLE OF INVENTION: Novel Alcohol/Aldehyde Dehydrogenases (iii) NUMBER OF SEQUENCES: 12 (iv) COMPUTER READABLE FORM:

(A) MEDIUM TYPE: Floppy disk
(B) COMPUTER: Macintosh
(C) OPERATING SYSTEM: Macintosh
(D) SOFTWARE: MS word ver 5.1

(v) CURRENT APPLICATION DATA:

(A) APPLICATION NUMBER: 97115801.9

(2) INFORMATION FOR SEQ ID NO:1:

(i) SEQUENCE CHARACTERISTICS:

(A) LENGTH: 1740
(B) TYPE: nucleic acid
(C) STRANDEDNESS: double
(D) TOPOLOGY: linear

(ix) SEQUENCE DESCRIPTION: SEQ ID NO:1

```
ATGAAACCGA CTTCGCTGCT TTGGGCCAGT GCTGGCGCAC TTGCATTGCT    50
TGCCGCACCC GCCTTTGCTC AAGTGACCCC CGTCACCGAT GAATTGCTGG   100
CGAACCCGCC CGCTGGTGAA TGGATCAGCT ACGGTCAGAA CCAAGAAAAC   150
TACCGTCACT CGCCCCTGAC GCAGATCACG ACTGAGAACG TCGGCCAACT   200
GCAACTGGTC TGGGCGCGCG GCATGCAGCC GGGCAAAGTC CAAGTCACGC   250
CCCTGATCCA TGACGGCGTC ATGTATCTGG CAAACCCGGG CGACGTGATC   300
CAGGCCATCG ACGCCAAAAC TGGCGATCTG ATCTGGGAAC ACCGCCGCCA   350
```

```
ACTGCCGAAC ATCGCCACGC TGAACAGCTT TGGCGAGCCG ACCCGCGGCA  400
TGGCGCTGTA CGGCACCAAC GTTTACTTTG TTTCGTGGGA CAACCACCTG  450
GTCGCCCTCG ACACCGCAAC TGGCCAAGTG ACGTTCGACG TCGACCGCGG  500
CCAAGGCGAA GACATGGTTT CGAACTCGTC GGGCCCGATC GTGGCAAACG  550
GCGTGATCGT TGCCGGTTCG ACCTGCCAAT ACTCGCCGTT CGGCTGCTTT  600
GTCTCGGGCC ACGACTCGGC CACCGGTGAA GAGCTGTGGC GCAACTACTT  650
CATCCCGCGC GCTGGCGAAG AGGGTGATGA GACTTGGGGC AACGATTACG  700
AAGCCCGTTG GATGACCGGT GCCTGGGGCC AGATCACCTA TGACCCCGTC  750
ACCAACCTTG TCCACTACGG CTCGACCGCT GTGGGTCCGG CGTCGGAAAC  800
CCAACGCGGC ACCCCGGGCG GCACGCTGTA CGGCACGAAC ACCCGTTTCG  850
CGGTGCGTCC TGACACGGGC GAGATTGTCT GGCGTCACCA GACCCTGCCC  900
CGCGACAACT GGGACCAGGA ATGCACGTTC GAGATGATGG TCACCAATGT  950
GGATGTCCAA CCCTCGACCG AGATGGAAGG TCTGCAGTCG ATCAACCCGA 1000
ACGCCGCAAC TGGCGAGCGT CGCGTGCTGA CCGGCGTTCC GTGCAAAACC 1050
GGCACCATGT GGCAGTTCGA CGCCGAAACC GGCGAATTCC TGTGGGCCCG 1100
TGATACCAAC TACCAGAACA TGATCGAATC CATCGACGAA AACGGCATCG 1150
TGACCGTGAA CGAAGATGCG ATCCTGAAGG AACTGGATGT TGAATATGAC 1200
GTCTGCCCGA CCTTCTTGGG CGGCCGCGAC TGGCCGTCGG CCGCACTGAA 1250
CCCCGACAGC GGCATCTACT TCATCCCGCT GAACAACGTC TGCTATGACA 1300
TGATGGCCGT CGATCAGGAA TTCACCTCGA TGGACGTCTA TAACACCAGC 1350
AACGTGACCA AGCTGCCGCC CGGCAAGGAT ATGATCGGTC GTATTGACGC 1400
GATCGACATC AGCACGGGTC GTACGCTGTG GTCGGTCGAA CGTGCTGCGG 1450
CGAACTATTC GCCCGTCTTG TCGACCGGCG GCGGCGTTCT GTTCAACGGT 1500
GGTACGGATC GTTACTTCCG CGCCCTCAGC CAAGAAACCG GCGAGACCCT 1550
GTGGCAGACC CGCCTTGCAA CCGTCGCGTC GGGCCAGGCC ATCTCTTACG 1600
AGGTTGACGG CATGCAATAT GTCGCCATCG CAGGTGGTGG TGTCAGCTAT 1650
GGCTCGGGCC TGAACTCGGC ACTGGCTGGC GAGCGAGTCG ACTCGACCGC 1700
CATCGGTAAC GCCGTCTACG TCTTCGCCCT GCCGCAATAA            1740
```

INFORMATION FOR SEQ ID NO:2:

(i) SEQUENCE CHARACTERISTICS:

(A) LENGTH: 1740
(B) TYPE: nucleic acid
(C) STRANDEDNESS: double
(D) TOPOLOGY: linear

(ix) SEQUENCE DESCRIPTION: SEQ ID NO:2

```
ATGAAGACGT CGTCTTTGCT GGTTGCGAGC GTTGCCGCGC TTGCAAGCTA   50
TAGCTCCTTT GCGCTTGCTC AAGTGACCCC CGTCACCGAT GAATTGCTGG  100
CGAACCCGCC CGCTGGTGAA TGGATCAGCT ACGGTCAGAA CCAAGAAAAC  150
TACCGTCACT CGCCCCTGAC GCAGATCACG ACTGAGAACG TCGGCCAACT  200
GCAACTGGTC TGGGCGCGCG GCATGCAGCC GGGCAAAGTC CAAGTCACGC  250
CCCTGATCCA TGACGGCGTC ATGTATCTGG CAAACCCGGG CGACGTGATC  300
CAGGCCATCG ACGCCAAAAC TGGCGATCTG ATCTGGGAAC ACCGCCGCCA  350
ACTGCCGAAC ATCGCCACGC TGAACAGCTT TGGCGAGCCG ACCCGCGGCA  400
TGGCGCTGTA CGGCACCAAC GTTTACTTTG TTTCGTGGGA CAACCACCTG  450
GTCGCCCTCG ACACCGCAAC TGGCCAAGTG ACGTTCGACG TCGACCGCGG  500
CCAAGGCGAA GACATGGTTT CGAACTCGTC GGGCCCGATC GTGGCAAACG  550
GCGTGATCGT TGCCGGTTCG ACCTGCCAAT ACTCGCCGTT CGGCTGCTTT  600
GTCTCGGGCC ACGACTCGGC CACCGGTGAA GAGCTGTGGC GCAACTACTT  650
CATCCCGCGC GCTGGCGAAG AGGGTGATGA GACTTGGGGC AACGATTACG  700
AAGCCCGTTG GATGACCGGC GTCTGGGGTC AGATCACCTA TGACCCCGTT  750
GGCGGCCTTG TCCACTACGG CTCGTCGGCT GTTGGCCCGG CTTCGGAAAC  800
CCAGCGCGGC ACCACCGGCG GCACCATGTA CGGCACCAAC ACCCGTTTCG  850
CTGTCCGTCC CGAGACTGGC GAGATCGTCT GGCGTCACCA AACTCTGCCC  900
CGCGACAACT GGGACCAAGA GTGCACCTTC GAGATGATGG TTGCCAACGT  950
TGACGTGCAG CCCGCAGCTG ACATGGACGG CGTCCGCTCG ATCAACCCGA 1000
ACGCCGCCAC CGGCGAGCGT CGCGTTCTGA CCGGCGTTCC GTGCAAAACC 1050
GGCACCATGT GGCAGTTCGA CGCCGAAACC GGCGAATTCC TGTGGGCCCG 1100
TGACACCAGC TACGAGAACA TCATCGAATC GATCGACGAA AACGGCATCG 1150
```

```
TGACCGTCGA CGAGTCGAAA GTTCTGACCG AGCTGGACAC CCCCTATGAC 1200
GTCTGCCCGC TGCTGCTGGG TGGCCGTGAC TGGCCGTCGG CTGCGCTGAA 1250
CCCCGATACC GGCATCTACT TTATCCCGCT GAACAACACC TGCATGGATA 1300
TCGAAGCTGT CGACCAGGAA TTCAGCTCGC TGGACGTGTA CAACCAAAGC 1350
CTGACCGCCA AAATGGCACC GGGTAAAGAG CTGGTTGGCC GTATCGACGC 1400
CATCGACATC AGCACAGGCC GCACCCTGTG GACCGCTGAG CGCGAAGCCT 1450
CGAACTACGC GCCTGTCCTG TCGACCGCTG GCGGCGTTCT GTTCAACGGC 1500
GGCACCGACC GTTACTTCCG CGCTCTCAGC CAAGAGACCG GCGAGACCCT 1550
GTGGCAGACC CGTCTGGCGA CTGTCGCTTC GGGCCAAGCT GTCTCGTACG 1600
AGATCGACGG CGTCCAATAC ATCGCCATCG GCGGCGGCGG CACGACCTAT 1650
GGTTCGTTCC ACAACCGTCC CCTGGCCGAG CCGGTCGACT CGACCGCGAT 1700
CGGTAATGCG ATGTACGTCT TCGCGCTGCC CCAGCAATAA            1740
```

INFORMATION FOR SEQ ID NO:3:

(i) SEQUENCE CHARACTERISTICS:

(A) LENGTH: 1737
(B) TYPE: nucleic acid
(C) STRANDEDNESS: double
(D) TOPOLOGY: linear

(ix) SEQUENCE DESCRIPTION: SEQ ID NO:3

```
ATGAAACTGA CGACCCTGCT GCAAAGCAGC GCCGCCCTGC TTGTGCTTGG   50
CACCATTCCC GCCCTTGCCC AAACCGCCAT CACCGATGAA ATGCTGGCGA  100
ACCCGCCCGC TGGTGAATGG ATCAACTACG GTCAGAACCA AGAGAACTAC  150
CGCCACTCGC CCCTGACGCA GATTACCGCA GACAACGTCG GCCAACTGCA  200
ACTGGTCTGG GCGCGCGGTA TGGAAGCGGG CAAGATCCAA GTGACCCCGC  250
TTGTCCATGA CGGCGTCATG TATCTGGCAA ACCCCGGTGA CGTGATCCAG  300
GCCATCGACG CCGCGACCGG CGATCTGATC TGGGAACACC GCCGCCAACT  350
GCCGAACATC GCCACGCTGA ACAGCTTTGG TGAGCCGACC CGCGGCATGG  400
CCCTCTATGG CACCAACGTC TATTTCGTCT CGTGGGACAA CCACTTGGTC  450
GCGCTGGACA CCTCGACCGG CCAAGTCGTA TTCGACGTCG ATCGCGGTCA  500
```

AGGCACGGAT ATGGTCTCGA ACTCGTCCGG CCCGATTGTC GCCAATGGCG 550
TCATCGTTGC GGGCTCGACC TGTCAGTATT CGCCGTTCGG CTGTTTCGTT 600
TCGGGCCACG ACTCGGCCAC CGGTGAAGAG CTGTGGCGCA ACAACTTTAT 650
CCCGCGCGCC GGCGAAGAGG GTGATGAGAC CTGGGGCAAT GATTACGAGG 700
CCCGCTGGAT GACCGGCGTT TGGGGCCAGA TCACCTATGA CCCCGTTGGC 750
GGCCTTGTCC ACTACGGCAC CTCAGCAGTT GGCCCTGCGG CCGAGATTCA 800
GCGCGGCACC GTTGGCGGCT CGATGTATGG CACCAACACC CGCTTTGCTG 850
TCCGCCCCGA GACCGGCGAG ATCGTCTGGC GTCACCAAAC TCTGCCCCGC 900
GACAACTGGG ACCAAGAGTG TACGTTCGAG ATGATGGTCG TCAACGTCGA 950
CGTCCAGCCC TCGGCTGAGA TGGAAGGCCT GCACGCCATC AACCCCGATG 1000
CCGCCACGGG CGAGCGTCGC GTTGTGACCG GCGTTCCGTG CAAGAACGGC 1050
ACCATGTGGC AGTTCGACGC CGAAACCGGC GAATTCCTGT GGGCGCGCGA 1100
CACCAGCTAT CAGAACCTGA TCGAAAGCGT CGATCCCGAT GGTCTGGTGC 1150
ATGTGAACGA AGATCTGGTC GTGACCGAGC TGGAAGTGGC CTATGAAATC 1200
TGCCCGACCT TCCTGGGTGG CCGCGACTGG CCGTCGGCTG CGCTGAACCC 1250
CGATACTGGC ATCTATTTCA TCCCGCTGAA CAACGCCTGT AGCGGTATGA 1300
CGGCTGTCGA CCAAGAGTTC AGCTCGCTCG ATGTGTATAA CGTCAGCCTC 1350
GACTATAAAC TGTCGCCCGG TTCGGAAAAC ATGGGCCGTA TCGACGCCAT 1400
CGACATCAGC ACCGGCCGCA CGCTGTGGTC GGCTGAACGC TACGCCTCGA 1450
ACTACGCGCC TGTCCTGTCC ACCGGCGGCG GCGTGCTGTT CAACGGCGGC 1500
ACCGACCGTT ACTTCCGCGC CCTCAGCCAA GAGACCGGCG AGACGCTGTG 1550
GCAGACCCGT CTGGCGACTG TCGCCTCGGG TCAAGCGATT TCCTATGAGA 1600
TCGACGGCGT GCAATATGTC GCCATCGGGC GCGGCGGCAC CAGCTATGGC 1650
AGCAACCACA ACCGCGCCCT GACCGAGCGG ATCGACTCGA CCGCCATCGG 1700
CAGCGCGATC TATGTCTTTG CTCTGCCGCA GCAGTAA 1737

INFORMATION FOR SEQ ID NO:4:

(i) SEQUENCE CHARACTERISTICS:

(A) LENGTH: 1740

(B) TYPE: nucleic acid
(C) STRANDEDNESS: double
(D) TOPOLOGY: linear

(ix) SEQUENCE DESCRIPTION: SEQ ID NO:4

```
ATGAACCCCA CAACGCTGCT TCGCACCAGC GCGGCCGTGC TATTGCTTAC   50
CGCGCCCGCC GCATTCGCGC AGGTAACCCC GATTACCGAT GAACTGCTGG  100
CGAACCCGCC CGCTGGTGAA TGGATTAACT ACGGCCGCAA CCAAGAAAAC  150
TATCGCCACT CGCCCCTGAC CCAGATCACT GCCGACAACG TTGGTCAGTT  200
GCAACTGGTC TGGGCCCGCG GGATGGAGGC GGGGGCCGTA CAGGTCACGC  250
CGATGATCCA TGATGGCGTG ATGTATCTGG CAAACCCCGG TGATGTGATC  300
CAGGCGCTGG ATGCGCAAAC AGGCGATCTG ATCTGGGAAC ACCGCCGCCA  350
ACTGCCCGCC GTCGCCACGC TAAACGCCCA AGGCGACCGC AAGCGCGGCG  400
TCGCCCTTTA CGGCACGAGC CTCTATTTCA GCTCATGGGA CAACCATCTG  450
ATCGCGCTGG ATATGGAGAC GGGCCAGGTC GTATTCGATG TCGAACGTGG  500
ATCGGGCGAA GACGGCTTGA CCAGTAACAC CACGGGGCCG ATTGTCGCCA  550
ATGGCGTCAT CGTCGCGGGT TCCACCTGCC AATATTCGCC CTATGGATGC  600
TTTATCTCGG GGCACGATTC CGCGACGGGT GAGGAGCTGT GGCGCAACCA  650
CTTTATCCCG CAGCCGGGCG AAGAGGGTGA CGAGACTTGG GGCAATGATT  700
TCGAGGCGCG CTGGATGACC GGCGTCTGGG GTCAGATCAC CTATGATCCC  750
GTGACGAACC TTGTGTTCTA TGGCTCGACC GGCGTGGGCC CAGCGTCCGA  800
AACCCAGCGC GGCACGCCGG GCGGCACGCT GTATGGCACC AACACCCGCT  850
TTGCGGTGCG TCCCGACACG GGCGAGATTG TCTGGCGTCA CCAGACCCTG  900
CCGCGCGACA ACTGGGACCA AGAATGCACG TTCGAGATGA TGGTCGCCAA  950
CGTCGATGTG CAACCCTCGG CCGAGATGGA GGGTCTGCGC GCCATCAACC 1000
CCAATGCGGC GACGGGCGAG CGCCGTGTGC TGACGGGTGC GCCTTGCAAG 1050
ACCGGCACGA TGTGGTCGTT TGATGCGGCC TCGGGCGAAT TCCTGTGGGC 1100
GCGTGATACC AACTACACCA ATATGATCGC CTCGATCGAC GAGACCGGCC 1150
```

```
TTGTGACGGT GAACGAGGAT GCGGTGCTGA AAGAGCTGGA CGTTGAATAT 1200
GACGTCTGCC CGACCTTCCT GGGTGGGCGC GACTGGTCGT CAGCCGCACT 1250
GAACCCGGAC ACCGGCATTT ACTTCTTGCC GCTGAACAAT GCCTGCTACG 1300
ATATTATGGC CGTTGATCAA GAGTTTAGCG CGCTCGACGT CTATAACACC 1350
AGCGCGACCG CAAAACTCGC GCCGGGCTTT GAAAATATGG GCCGCATCGA 1400
CGCGATTGAT ATCAGCACCG GGCGCACCTT GTGGTCGGCG GAGCGCCCTG 1450
CGGCGAACTA CTCGCCCGTT TTGTCGACGG CAGGCGGTGT GGTGTTCAAC 1500
GGCGGGACCG ACCGCTATTT CCGTGCCCTC AGCCAGGAAA CCGGCGAGAC 1550
TTTGTGGCAG GCCCGTCTTG CGACGGTCGC GACGGGGCAG GCGATCAGCT 1600
ACGAGTTGGA CGGCGTGCAA TATATCGCCA TCGGTGCGGG CGGTCTGACC 1650
TATGGCACGC AATTGAACGC GCCGCTGGCC GAGGCAATCG ATTCGACCTC 1700
GGTCGGTAAT GCGATCTATG TCTTTGCACT GCCGCAGTAA            1740
```

INFORMATION FOR SEQ ID NO:5:

(i) SEQUENCE CHARACTERISTICS:

(A) LENGTH: 579
(B) TYPE: amino acid
(C) STRANDEDNESS: single
(D) TOPOLOGY: linear

(ix) SEQUENCE DESCRIPTION: SEQ ID NO:5

```
Met Lys Pro Thr Ser Leu Leu Trp Ala Ser Ala Gly Ala Leu Ala
            -20                 -15                 -10

Leu Leu Ala Ala Pro Ala Phe Ala Gln Val Thr Pro Val Thr Asp
            -5                  1                   5

Glu Leu Leu Ala Asn Pro Pro Ala Gly Glu Trp Ile Ser Tyr Gly
        10                  15                  20

Gln Asn Gln Glu Asn Tyr Arg His Ser Pro Leu Thr Gln Ile Thr
        25                  30                  35

Thr Glu Asn Val Gly Gln Leu Gln Leu Val Trp Ala Arg Gly Met
        40                  45                  50

Gln Pro Gly Lys Val Gln Val Thr Pro Leu Ile His Asp Gly Val
        55                  60                  65

Met Tyr Leu Ala Asn Pro Gly Asp Val Ile Gln Ala Ile Asp Ala
        70                  75                  80
```

Lys Thr Gly Asp Leu Ile Trp Glu His Arg Arg Gln Leu Pro Asn
85 90 95

Ile Ala Thr Leu Asn Ser Phe Gly Glu Pro Thr Arg Gly Met Ala
100 105 110

Leu Tyr Gly Thr Asn Val Tyr Phe Val Ser Trp Asp Asn His Leu
115 120 125

Val Ala Leu Asp Thr Ala Thr Gly Gln Val Thr Phe Asp Val Asp
130 135 140

Arg Gly Gln Gly Glu Asp Met Val Ser Asn Ser Ser Gly Pro Ile
145 150 155

Val Ala Asn Gly Val Ile Val Ala Gly Ser Thr Cys Gln Tyr Ser
160 165 170

Pro Phe Gly Cys Phe Val Ser Gly His Asp Ser Ala Thr Gly Glu
175 180 185

Glu Leu Trp Arg Asn Tyr Phe Ile Pro Arg Ala Gly Glu Glu Gly
190 195 200

Asp Glu Thr Trp Gly Asn Asp Tyr Glu Ala Arg Trp Met Thr Gly
205 210 215

Ala Trp Gly Gln Ile Thr Tyr Asp Pro Val Thr Asn Leu Val His
220 225 230

Tyr Gly Ser Thr Ala Val Gly Pro Ala Ser Glu Thr Gln Arg Gly
235 240 245

Thr Pro Gly Gly Thr Leu Tyr Gly Thr Asn Thr Arg Phe Ala Val
250 255 260

Arg Pro Asp Thr Gly Glu Ile Val Trp Arg His Gln Thr Leu Pro
265 270 275

Arg Asp Asn Trp Asp Gln Glu Cys Thr Phe Glu Met Met Val Thr
280 285 290

Asn Val Asp Val Gln Pro Ser Thr Glu Met Glu Gly Leu Gln Ser
295 300 305

Ile Asn Pro Asn Ala Ala Thr Gly Glu Arg Arg Val Leu Thr Gly
310 315 320

Val Pro Cys Lys Thr Gly Thr Met Trp Gln Phe Asp Ala Glu Thr
325 330 335

Gly Glu Phe Leu Trp Ala Arg Asp Thr Asn Tyr Gln Asn Met Ile
340 345 350

Glu Ser Ile Asp Glu Asn Gly Ile Val Thr Val Asn Glu Asp Ala
355 360 365

```
Ile Leu Lys Glu Leu Asp Val Glu Tyr Asp Val Cys Pro Thr Phe
        370                 375                 380

Leu Gly Gly Arg Asp Trp Pro Ser Ala Ala Leu Asn Pro Asp Ser
        385                 390                 395

Gly Ile Tyr Phe Ile Pro Leu Asn Asn Val Cys Tyr Asp Met Met
        400                 405                 410

Ala Val Asp Gln Glu Phe Thr Ser Met Asp Val Tyr Asn Thr Ser
        415                 420                 425

Asn Val Thr Lys Leu Pro Pro Gly Lys Asp Met Ile Gly Arg Ile
        430                 435                 440

Asp Ala Ile Asp Ile Ser Thr Gly Arg Thr Leu Trp Ser Val Glu
        445                 450                 455

Arg Ala Ala Ala Asn Tyr Ser Pro Val Leu Ser Thr Gly Gly Gly
        460                 465                 470

Val Leu Phe Asn Gly Gly Thr Asp Arg Tyr Phe Arg Ala Leu Ser
        475                 480                 485

Gln Glu Thr Gly Glu Thr Leu Trp Gln Thr Arg Leu Ala Thr Val
        490                 495                 500

Ala Ser Gly Gln Ala Ile Ser Tyr Glu Val Asp Gly Met Gln Tyr
        505                 510                 515

Val Ala Ile Ala Gly Gly Gly Val Ser Tyr Gly Ser Gly Leu Asn
        520                 525                 530

Ser Ala Leu Ala Gly Glu Arg Val Asp Ser Thr Ala Ile Gly Asn
        535                 540                 545

Ala Val Tyr Val Phe Ala Leu Pro Gln
        550                 555
```

INFORMATION FOR SEQ ID NO:6:

(i) SEQUENCE CHARACTERISTICS:

(A) LENGTH: 579
(B) TYPE: amino acid
(C) STRANDEDNESS: single
(D) TOPOLOGY: linear

(ix) SEQUENCE DESCRIPTION: SEQ ID NO:6

```
Met Lys Thr Ser Ser Leu Leu Val Ala Ser Val Ala Ala Leu Ala
            -20                 -15                 -10

Ser Tyr Ser Ser Phe Ala Leu Ala Gln Val Thr Pro Val Thr Asp
            -5                  1               5
```

```
Glu Leu Leu Ala Asn Pro Pro Ala Gly Glu Trp Ile Ser Tyr Gly
        10                  15                  20

Gln Asn Gln Glu Asn Tyr Arg His Ser Pro Leu Thr Gln Ile Thr
        25                  30                  35

Thr Glu Asn Val Gly Gln Leu Gln Leu Val Trp Ala Arg Gly Met
        40                  45                  50

Gln Pro Gly Lys Val Gln Val Thr Pro Leu Ile His Asp Gly Val
        55                  60                  65

Met Tyr Leu Ala Asn Pro Gly Asp Val Ile Gln Ala Ile Asp Ala
        70                  75                  80

Lys Thr Gly Asp Leu Ile Trp Glu His Arg Arg Gln Leu Pro Asn
        85                  90                  95

Ile Ala Thr Leu Asn Ser Phe Gly Glu Pro Thr Arg Gly Met Ala
        100                 105                 110

Leu Tyr Gly Thr Asn Val Tyr Phe Val Ser Trp Asp Asn His Leu
        115                 120                 125

Val Ala Leu Asp Thr Ala Thr Gly Gln Val Thr Phe Asp Val Asp
        130                 135                 140

Arg Gly Gln Gly Glu Asp Met Val Ser Asn Ser Ser Gly Pro Ile
        145                 150                 155

Val Ala Asn Gly Val Ile Val Ala Gly Ser Thr Cys Gln Tyr Ser
        160                 165                 170

Pro Phe Gly Cys Phe Val Ser Gly His Asp Ser Ala Thr Gly Glu
        175                 180                 185

Glu Leu Trp Arg Asn Tyr Phe Ile Pro Arg Ala Gly Glu Glu Gly
        190                 195                 200

Asp Glu Thr Trp Gly Asn Asp Tyr Glu Ala Arg Trp Met Thr Gly
        205                 210                 215

Val Trp Gly Gln Ile Thr Tyr Asp Pro Val Gly Gly Leu Val His
        220                 225                 230

Tyr Gly Ser Ser Ala Val Gly Pro Ala Ser Glu Thr Gln Arg Gly
        235                 240                 245

Thr Thr Gly Gly Thr Met Tyr Gly Thr Asn Thr Arg Phe Ala Val
        250                 255                 260

Arg Pro Glu Thr Gly Glu Ile Val Trp Arg His Gln Thr Leu Pro
        265                 270                 275

Arg Asp Asn Trp Asp Gln Glu Cys Thr Phe Glu Met Met Val Ala
        280                 285                 290
```

Asn Val Asp Val Gln Pro Ala Ala Asp Met Asp Gly Val Arg Ser
295 300 305

Ile Asn Pro Asn Ala Ala Thr Gly Glu Arg Arg Val Leu Thr Gly
310 315 320

Val Pro Cys Lys Thr Gly Thr Met Trp Gln Phe Asp Ala Glu Thr
325 330 335

Gly Glu Phe Leu Trp Ala Arg Asp Thr Ser Tyr Glu Asn Ile Ile
340 345 350

Glu Ser Ile Asp Glu Asn Gly Ile Val Thr Val Asp Glu Ser Lys
355 360 365

Val Leu Thr Glu Leu Asp Thr Pro Tyr Asp Val Cys Pro Leu Leu
370 375 380

Leu Gly Gly Arg Asp Trp Pro Ser Ala Ala Leu Asn Pro Asp Thr
385 390 395

Gly Ile Tyr Phe Ile Pro Leu Asn Asn Thr Cys Met Asp Ile Glu
400 405 410

Ala Val Asp Gln Glu Phe Ser Ser Leu Asp Val Tyr Asn Gln Ser
415 420 425

Leu Thr Ala Lys Met Ala Pro Gly Lys Glu Leu Val Gly Arg Ile
430 435 440

Asp Ala Ile Asp Ile Ser Thr Gly Arg Thr Leu Trp Thr Ala Glu
445 450 455

Arg Glu Ala Ser Asn Tyr Ala Pro Val Leu Ser Thr Ala Gly Gly
460 465 470

Val Leu Phe Asn Gly Gly Thr Asp Arg Tyr Phe Arg Ala Leu Ser
475 480 485

Gln Glu Thr Gly Glu Thr Leu Trp Gln Thr Arg Leu Ala Thr Val
490 495 500

Ala Ser Gly Gln Ala Val Ser Tyr Glu Ile Asp Gly Val Gln Tyr
505 510 515

Ile Ala Ile Gly Gly Gly Gly Thr Thr Tyr Gly Ser Phe His Asn
520 525 530

Arg Pro Leu Ala Glu Pro Val Asp Ser Thr Ala Ile Gly Asn Ala
535 540 545

Met Tyr Val Phe Ala Leu Pro Gln Gln
550 555

INFORMATION FOR SEQ ID NO:7:

(i) SEQUENCE CHARACTERISTICS:

(A) LENGTH: 578
(B) TYPE: amino acid
(C) STRANDEDNESS: single
(D) TOPOLOGY: linear

(ix) SEQUENCE DESCRIPTION: SEQ ID NO:7

```
Met Lys Leu Thr Thr Leu Leu Gln Ser Ser Ala Ala Leu Leu Val
            -20                 -15                 -10

Leu Gly Thr Ile Pro Ala Leu Ala Gln Thr Ala Ile Thr Asp Glu
             -5                   1                   5

Met Leu Ala Asn Pro Pro Ala Gly Glu Trp Ile Asn Tyr Gly Gln
         10                  15                  20

Asn Gln Glu Asn Tyr Arg His Ser Pro Leu Thr Gln Ile Thr Ala
         25                  30                  35

Asp Asn Val Gly Gln Leu Gln Leu Val Trp Ala Arg Gly Met Glu
         40                  45                  50

Ala Gly Lys Ile Gln Val Thr Pro Leu Val His Asp Gly Val Met
         55                  60                  65

Tyr Leu Ala Asn Pro Gly Asp Val Ile Gln Ala Ile Asp Ala Ala
         70                  75                  80

Thr Gly Asp Leu Ile Trp Glu His Arg Arg Gln Leu Pro Asn Ile
         85                  90                  95

Ala Thr Leu Asn Ser Phe Gly Glu Pro Thr Arg Gly Met Ala Leu
        100                 105                 110

Tyr Gly Thr Asn Val Tyr Phe Val Ser Trp Asp Asn His Leu Val
        115                 120                 125

Ala Leu Asp Thr Ser Thr Gly Gln Val Val Phe Asp Val Asp Arg
        130                 135                 140

Gly Gln Gly Thr Asp Met Val Ser Asn Ser Ser Gly Pro Ile Val
        145                 150                 155

Ala Asn Gly Val Ile Val Ala Gly Ser Thr Cys Gln Tyr Ser Pro
        160                 165                 170

Phe Gly Cys Phe Val Ser Gly His Asp Ser Ala Thr Gly Glu Glu
        175                 180                 185

Leu Trp Arg Asn Asn Phe Ile Pro Arg Ala Gly Glu Glu Gly Asp
        190                 195                 200
```

```
Glu Thr Trp Gly Asn Asp Tyr Glu Ala Arg Trp Met Thr Gly Val
        205                 210                 215

Trp Gly Gln Ile Thr Tyr Asp Pro Val Gly Gly Leu Val His Tyr
        220                 225                 230

Gly Thr Ser Ala Val Gly Pro Ala Ala Glu Ile Gln Arg Gly Thr
        235                 240                 245

Val Gly Gly Ser Met Tyr Gly Thr Asn Thr Arg Phe Ala Val Arg
        250                 255                 260

Pro Glu Thr Gly Glu Ile Val Trp Arg His Gln Thr Leu Pro Arg
        265                 270                 275

Asp Asn Trp Asp Gln Glu Cys Thr Phe Glu Met Met Val Val Asn
        280                 285                 290

Val Asp Val Gln Pro Ser Ala Glu Met Glu Gly Leu His Ala Ile
        295                 300                 305

Asn Pro Asp Ala Ala Thr Gly Glu Arg Arg Val Val Thr Gly Val
        310                 315                 320

Pro Cys Lys Asn Gly Thr Met Trp Gln Phe Asp Ala Glu Thr Gly
        325                 330                 335

Glu Phe Leu Trp Ala Arg Asp Thr Ser Tyr Gln Asn Leu Ile Glu
        340                 345                 350

Ser Val Asp Pro Asp Gly Leu Val His Val Asn Glu Asp Leu Val
        355                 360                 365

Val Thr Glu Leu Glu Val Ala Tyr Glu Ile Cys Pro Thr Phe Leu
        370                 375                 380

Gly Gly Arg Asp Trp Pro Ser Ala Ala Leu Asn Pro Asp Thr Gly
        385                 390                 395

Ile Tyr Phe Ile Pro Leu Asn Asn Ala Cys Ser Gly Met Thr Ala
        400                 405                 410

Val Asp Gln Glu Phe Ser Ser Leu Asp Val Tyr Asn Val Ser Leu
        415                 420                 425

Asp Tyr Lys Leu Ser Pro Gly Ser Glu Asn Met Gly Arg Ile Asp
        430                 435                 440

Ala Ile Asp Ile Ser Thr Gly Arg Thr Leu Trp Ser Ala Glu Arg
        445                 450                 455

Tyr Ala Ser Asn Tyr Ala Pro Val Leu Ser Thr Gly Gly Gly Val
        460                 465                 470

Leu Phe Asn Gly Gly Thr Asp Arg Tyr Phe Arg Ala Leu Ser Gln
        475                 480                 485
```

```
Glu Thr Gly Glu Thr Leu Trp Gln Thr Arg Leu Ala Thr Val Ala
        490                 495                 500

Ser Gly Gln Ala Ile Ser Tyr Glu Ile Asp Gly Val Gln Tyr Val
        505                 510                 515

Ala Ile Gly Arg Gly Gly Thr Ser Tyr Gly Ser Asn His Asn Arg
        520                 525                 530

Ala Leu Thr Glu Arg Ile Asp Ser Thr Ala Ile Gly Ser Ala Ile
        535                 540                 545

Tyr Val Phe Ala Leu Pro Gln Gln
        550                 555
```

INFORMATION FOR SEQ ID NO:8:

(i) SEQUENCE CHARACTERISTICS:

(A) LENGTH: 579
(B) TYPE: amino acid
(C) STRANDEDNESS: single
(D) TOPOLOGY: linear

(ix) SEQUENCE DESCRIPTION: SEQ ID NO:8

```
Met Asn Pro Thr Thr Leu Leu Arg Thr Ser Ala Ala Val Leu Leu
            -20                 -15                 -10

Leu Thr Ala Pro Ala Ala Phe Ala Gln Val Thr Pro Ile Thr Asp
            -5                   1               5

Glu Leu Leu Ala Asn Pro Pro Ala Gly Glu Trp Ile Asn Tyr Gly
        10                  15                  20

Arg Asn Gln Glu Asn Tyr Arg His Ser Pro Leu Thr Gln Ile Thr
        25                  30                  35

Ala Asp Asn Val Gly Gln Leu Gln Leu Val Trp Ala Arg Gly Met
        40                  45                  50

Glu Ala Gly Ala Val Gln Val Thr Pro Met Ile His Asp Gly Val
        55                  60                  65

Met Tyr Leu Ala Asn Pro Gly Asp Val Ile Gln Ala Leu Asp Ala
        70                  75                  80

Gln Thr Gly Asp Leu Ile Trp Glu His Arg Arg Gln Leu Pro Ala
        85                  90                  95

Val Ala Thr Leu Asn Ala Gln Gly Asp Arg Lys Arg Gly Val Ala
        100                 105                 110

Leu Tyr Gly Thr Ser Leu Tyr Phe Ser Ser Trp Asp Asn His Leu
        115                 120                 125
```

```
Ile Ala Leu Asp Met Glu Thr Gly Gln Val Val Phe Asp Val Glu
        130                 135                 140

Arg Gly Ser Gly Glu Asp Gly Leu Thr Ser Asn Thr Thr Gly Pro
        145                 150                 155

Ile Val Ala Asn Gly Val Ile Val Ala Gly Ser Thr Cys Gln Tyr
        160                 165                 170

Ser Pro Tyr Gly Cys Phe Ile Ser Gly His Asp Ser Ala Thr Gly
        175                 180                 185

Glu Glu Leu Trp Arg Asn His Phe Ile Pro Gln Pro Gly Glu Glu
        190                 195                 200

Gly Asp Glu Thr Trp Gly Asn Asp Phe Glu Ala Arg Trp Met Thr
        205                 210                 215

Gly Val Trp Gly Gln Ile Thr Tyr Asp Pro Val Thr Asn Leu Val
        220                 225                 230

Phe Tyr Gly Ser Thr Gly Val Gly Pro Ala Ser Glu Thr Gln Arg
        235                 240                 245

Gly Thr Pro Gly Gly Thr Leu Tyr Gly Thr Asn Thr Arg Phe Ala
        250                 255                 260

Val Arg Pro Asp Thr Gly Glu Ile Val Trp Arg His Gln Thr Leu
        265                 270                 275

Pro Arg Asp Asn Trp Asp Gln Glu Cys Thr Phe Glu Met Met Val
        280                 285                 290

Ala Asn Val Asp Val Gln Pro Ser Ala Glu Met Glu Gly Leu Arg
        295                 300                 305

Ala Ile Asn Pro Asn Ala Ala Thr Gly Glu Arg Arg Val Leu Thr
        310                 315                 320

Gly Ala Pro Cys Lys Thr Gly Thr Met Trp Ser Phe Asp Ala Ala
        325                 330                 335

Ser Gly Glu Phe Leu Trp Ala Arg Asp Thr Asn Tyr Thr Asn Met
        340                 345                 350
Ile Ala Ser Ile Asp Glu Thr Gly Leu Val Thr Val Asn Glu Asp
        355                 360                 365
Ala Val Leu Lys Glu Leu Asp Val Glu Tyr Asp Val Cys Pro Thr
        370                 375                 380
Phe Leu Gly Gly Arg Asp Trp Ser Ser Ala Ala Leu Asn Pro Asp
        385                 390                 395
Thr Gly Ile Tyr Phe Leu Pro Leu Asn Asn Ala Cys Tyr Asp Ile
        400                 405                 410
```

```
Met Ala Val Asp Gln Glu Phe Ser Ala Leu Asp Val Tyr Asn Thr
        415                 420                 425

Ser Ala Thr Ala Lys Leu Ala Pro Gly Phe Glu Asn Met Gly Arg
        430                 435                 440

Ile Asp Ala Ile Asp Ile Ser Thr Gly Arg Thr Leu Trp Ser Ala
        445                 450                 455

Glu Arg Pro Ala Ala Asn Tyr Ser Pro Val Leu Ser Thr Ala Gly
        460                 465                 470

Gly Val Val Phe Asn Gly Gly Thr Asp Arg Tyr Phe Arg Ala Leu
        475                 480                 485

Ser Gln Glu Thr Gly Glu Thr Leu Trp Gln Ala Arg Leu Ala Thr
        490                 495                 500

Val Ala Thr Gly Gln Ala Ile Ser Tyr Glu Leu Asp Gly Val Gln
        505                 510                 515

Tyr Ile Ala Ile Gly Ala Gly Gly Leu Thr Tyr Gly Thr Gln Leu
        520                 525                 530

Asn Ala Pro Leu Ala Glu Ala Ile Asp Ser Thr Ser Val Gly Asn
        535                 540                 545

Ala Ile Tyr Val Phe Ala Leu Pro Gln
        550                 555
```

INFORMATION FOR SEQ ID NO:9:

(i) SEQUENCE CHARACTERISTICS:

(A) LENGTH: 82
(B) TYPE: nucleotide
(C) STRANDEDNESS: single
(D) TOPOLOGY: linear

(ix) SEQUENCE DESCRIPTION: SEQ ID NO:9

```
CATGAAAATA AAAACAGGTG CACGCATCCT CGCATTATCC GCATTAACGA 50
CGATGATGTT TTCCGCCTCG GCTCTCGCCC AG                   82
```

INFORMATION FOR SEQ ID NO:10:

(i) SEQUENCE CHARACTERISTICS:

(A) LENGTH: 83
(B) TYPE: nucleotide

(C) STRANDEDNESS: single
(D) TOPOLOGY: linear

(ix) SEQUENCE DESCRIPTION: SEQ ID NO:10

```
GTTACCTGGG CGAGAGCCGA GGCGGAAAAC ATCATCGTCG TTAATGCGGA 50
TAATGCGAGG ATGCGTGCAC CTGTTTTTAT TTT                   83
```

INFORMATION FOR SEQ ID NO:11:

(i) SEQUENCE CHARACTERISTICS:

(A) LENGTH: 27
(B) TYPE: amino acid
(C) STRANDEDNESS: single
(D) TOPOLOGY: linear

(ix) SEQUENCE DESCRIPTION: SEQ ID NO:11

```
Met Lys Ile Lys Thr Gly Ala Arg Ile Leu Ala Leu Ser Ala Leu
 1               5                  10                  15

Thr Thr Met Met Phe Ser Ala Ser Ala Leu Ala Gln
                 20                 25      27
```

INFORMATION FOR SEQ ID NO:12:

(i) SEQUENCE CHARACTERISTICS:

(A) LENGTH: 27
(B) TYPE: nucleotide
(C) STRANDEDNESS: single
(D) TOPOLOGY: linear

(ix) SEQUENCE DESCRIPTION: SEQ ID NO:12
GTTAGCGCGG TGGATCCCCA TTGGAGG 27

## Description

The present invention relates to recombinant enzyme preparations of alcohol/aldehyde dehydrogenase(s) (hereinafter referred to as AADH or AADHs) having alcohol and/or aldehyde dehydrogenase activity (activities). The present invention also relates to novel recombinant DNA molecule(s) encoding AADH(s), recombinant expression vector (s) containing said DNA(s), and recombinant organism(s) containing said recombinant DNA molecule(s) and/or said recombinant expression vector(s). Furthermore, the present invention relates to a process for producing recombinant enzyme preparation(s) of AADH(s) and a process for producing aldehyde(s), carboxylic acid(s) and ketone(s), especially, 2-keto-L-gulonic acid (herein after referred to as 2KGA) by utilizing said recombinant enzyme preparation(s), and a process for producing aldehyde(s), carboxylic acid(s) and ketone(s), especially, 2KGA by utilizing said recombinant organism(s).

2-KGA is an important intermediate for the production of L-ascorbic acid (vitamin C). Numerous microorganisms are known to produce 2KGA from D-sorbitol or L-sorbose. Japanese Patent Publication No. 51-40154 (1976) discloses the production of 2KGA from D-sorbitol by microorganisms of the genus *Acetobacter, Bacterium* or *Pseudomonas*. According to Acta Microbiologica Sinica 21(2), 185 - 191 (1981), 2KGA can be produced from L-sorbose by a mixed culture of microorganisms, especially, *Pseudomonas striata* and *Gluconobacter oxydans*. European Patent Publication No. 0221 707 discloses the production of 2KGA from L-sorbose by *Pseudogluconobacter saccharoketogenes* with and without concomitant bacteria. European Patent Publication No. 0278 447 discloses a process for the production of 2KGA from L-sorbose by a mixed culture, which is composed of strain DSM No. 4025 (*Gluconobacter oxydans*) and DSM No. 4026 (*a Bacillus megaterium* strain). European Patent Publication No. 88116156 discloses a process for the production of 2KGA from L-sorbose by *Gluconobacter oxydans* DSM No. 4025.

From *G. oxydans* DSM No. 4025, AADH was purified and characterized to catalyze the oxidation of alcohols and aldehydes, and was thus capable of producing the corresponding aldehydes and ketones from alcohols, and carboxylic acids from aldehydes (seeEuropean Patent Publication No. 606621). More particularly, the AADH catalyzed the oxidation of L-sorbose to 2KGA via L-sorbosone. The physico-chemical properties of the purified sample of the AADH were as follows:

a) Optimum pH: about 7.0 - 9.0
b) Optimum temperature: about 20°C - 40°C
c) Molecular weight: 135,000 +/- 5,000 dalton (Consisting of two subunits in any combination of such $\alpha$-subunit and $\beta$-subunit, each having a molecular weight of 64,500 +/- 2,000 and 62,500 +/- 2,000, respectively)
d) Substrate specificity: active on primary and secondary alcohols and aldehydes including L-sorbose, L-sorbosone, D-sorbitol, D-glucose, D-mannitol, D-fructose, DL-glycelaldehyde, ethanol, 1-propanol, 1-butanol, 1-pentanol, 1-hexanol, 1-heptanol, 2-propanol, 2-butanol, propionaldehyde, PEG1000, PEG2000, PEG4000, PEG6000 and polyvinyl alcohol
e) Prosthetic group: pyrroloquinoline quinone
f) Isoelectric point: about 4.4

Once the gene(s) coding for said AADH have been cloned, they can be used for the construction of a recombinant organism capable of producing a large amount of the recombinant enzyme preparation of AADH or the various aldehydes, ketones and carboxylic acids, especially, 2KGA. However, there have been no reports so far of the cloning of such genes.

Therefore the present invention relates to novel recombinant enzyme preparation(s) of AADH(s) having alcohol and/or aldehyde dehydrogenase activity (activities). Comprised by the present invention are novel recombinant molecule (s) encoding the AADH(s); recombinant expression vector(s) containing said DNAs; recombinant organism(s) carrying said DNA(s) and/or recombinant expression vector(s); a process for producing the recombinant AADH(s); and a process for producing aldehyde(s), carboxylic acid(s) and ketone(s), especially, 2KGA utilizing the recombinant AADH(s) or the recombinant organism(s).

More particularly, an aspect of the present invention concerns a recombinant enzyme preparation having an alcohol and/or aldehyde dehydrogenase activity which comprises enzymatic polypeptide identified by SEQ ID NO 8 or an enzymatic polypeptide encoded by a DNA molecule according to SEQ ID NO: 4 or a DNA sequence which hybridizes under stringent hybridization and stringent washing conditions with said DNA molecule, said DNA encoding a polypeptide, wherein said enzymatic polypeptides have alcohol and/or aldehyde dehydrogenase activity.

Another aspect of the present invention concerns a recombinant DNA molecule encoding enzymatic polypeptide identified by SEQ ID NO 8 wherein said enzymatic polypeptides have alcohol and/or aldehyde dehydrogenase activity.

Furthermore the present invention is directed to DNA sequences encoding the polypeptides with alcohol and/or aldehyd dehydrogenase activity as disclosed e.g. in the sequence listing as well as their complementary strands those which include these sequences, DNA sequences which hybridize under stringent hybridization and stringent washing conditions with such sequences and DNA sequences, which because of the degeneration of the genetic code, do not hybridize under stringent hybridization and stringent washing conditions with such sequences but which code for polypeptides having exactly the same amino acid sequence.

"stringent hybridization and washing conditions" for hybridization mean in this context the conditions which are generally used by a man skilled in the art to detect specific hybridization signals and which are described, e.g. by Sambrook et al., "Molecular Cloning" second edition, Cold Spring Harbour Laboratory Press 1989, New York. Furthermore DNA sequences which can be made by the polymerase chain reaction by using primers designed on the basis of the DNA sequences disclosed herein by methods known in the art are also an object of the present invention. It is understood that the DNA sequences of the present invention can also be made synthetically as described, e.g. in EP 747 483.

Further aspects of the present invention concern a recombinant expression vector which carries one or more of the recombinant DNA molecule(s) defined above and a recombinant host cell which carries the recombinant expression vector defined above.

A further aspect of the present invention concerns a process for producing a recombinant enzyme preparation having an alcohol and/or aldehyde dehydrogenase activity as defined above, which comprises cultivating a recombinant host cell defined above in an appropriate culture medium and recovering said recombinant enzyme preparation.

Another aspect of the present invention concerns a process for producing an aldehyde, ketone or carboxylic acid product from a corresponding substrate which comprises converting said substrate into the product by the use of a recombinant host cell as defined above.

Moreover another aspect of the present invention concerns a process form producing 2-keto-L-gulonic acid which comprises the fermentation of a recombinant host cell as defined above in an appropriate medium containing L-sorbose and/or D-sorbitol.

Another aspect of the present invention concerns a process for producing an aldehyde, ketone or carboxylic acid product from a corresponding substrate which comprises the incubation of a reaction mixture containing a recombinant enzyme preparation of the present invention.

Further more another aspect of the present invention concerns a process for producing 2-keto-L-gulonic acid which comprises the incubation of a reaction mixture containing a recombinant enzyme preparation defined above and L-sorbose and/or D-sorbitol.

It is also an object of the present invention to provide a process for the production of vitamin C from 2-keto-L-gulonic acid characterized therein that a process for the production of 2-keto-L-gulonic acid as described above is effected and the 2-keto-L-gulonic acid obtained by such process is transformed into vitamin C (L-ascorbic acid) by methods known in the art.

Before describing the present invention in more detail a short explanation of the attached figures is given.

Figure 1 schematically illustrates the structures of the recombinant expression vectors each carrying the recombinant DNA molecule which encodes the recombinant Enzyme A or B of the present invention.
Figure 5 shows the alignment of the amino acid sequences of the mature Enzyme A and Enzyme B.
Figure 7 shows the restriction map of the genes of Enzymes A and B.
Figure 9 shows a site-directed mutagenesis to introduce a *Bam*HI site upstream of the Enzyme B gene.
Figure 10 illustrate a scheme of the replacement of the promoter for the Enzyme B gene.
Figure 11 shows graphs illustrating the substrate specificity of chimeric enzymes of the invention.

The AADH genes of the present invention encode the AADH enzymes capable of catalyzing the oxidation of various alcohols and aldehydes as described above. Specifically speaking the particular genes of AADHs present in *Gluconobacter* were cloned and expressed. Alternative sources in addition to *Gluconobacter* may well be found among the other organisms by the man skilled in the art using the teachings of the present invention.

A specific and preferred *Gluconobacter oxydans* strain has been deposited at the Deutsche Sammlung von Mikroorganismen in Göttingen (Germany) under DSM No. 4025.

Moreover, a subculture of the strain has also been deposited in the Agency of Industrial Science and Technology, Fermentation Research Institute, Japan, under the deposit No.: FERM BP-3812. European Patent publication No. 0278 477 disclose the characteristics of this strain.

The AADH genes and the recombinant microorganisms utilized in the present invention can be obtained by the following steps:

(1) Cloning the AADH genes from a chromosomal DNA by colony- or plaque-hybridization, PCR cloning, Western-blot analysis, Southern-blot hybridization and the like.

(2) Determining the nucleotide sequences of such AADH genes by usual methods and constructing recombinant expression vectors which contain and express AADH genes efficiently.

(3) Constructing recombinant microorganisms carrying recombinant AADH genes on recombinant expression vectors or on chromosomes by transformation, transduction, transconjugation and electroporation.

The materials and the techniques applicable to the above aspect of the present invention are exemplified in details as described in the following:

A total chromosomal DNA can be purified by a procedure well known in the art (Marmur J., J. Mol. Biol. 3:208, 1961). Then, a genomic library of the strain for such genes can be constructed with the chromosomal DNA and the vectors described below in detail. The genes encoding AADHs can be cloned in either plasmid or phage vectors from the total chromosomal DNA by the following methods:

(i) determining the partial amino acid sequences of the purified enzyme, according to the sequence information, synthesizing the oligonucleotides, and selecting the objective gene from the gene library by Southern-blot-, colony-, or plaque-hybridization;

(ii) by amplifying the partial sequence of the desired gene by polymerase chain reaction (PCR) with the oligonucleotides synthesized as described above as the primers and with the PCR product as a probe, selecting the complete sequence of the objective gene from the gene library by Southern-blot-, colony-, or plaque-hybridization;

(iii) by preparing the antibody reacting against the desired enzyme protein by such a method as previously described, e.g. in Methods in Enzymology, vol. 73, p 46, 1981, and selecting the clone which expresses the desired polypeptide by immnunological analysis including Western-blot analysis;

(iv) by aligning the amino acid sequences of the homologs to the one of the desired enzyme, selecting the amino acid sequences which are well conserved, synthesizing the oligonucleotides encoding the conserved sequences, amplifying the partial sequence of the desired gene by PCR with the above oligonucleotides as the primers, and selecting the complete sequence as described above (ii).

The nucleotide sequence of the desired gene can be determined by a well known method such as the dideoxy chain termination method with the M13 phage (Sanger F., et al., Proc. Natl. Acad. Sci.USA, 74:5463-5467, 1977).

By using the information of the so determined nucleotide sequence (in consideration of the codon usage) a gene encoding evolutionally divergent alcohol and/or aldehyde dehydrogenases, can be isolated from a different organism by colony- or Southern-hybridization with a probe synthesized according to the amino acid sequence deduced from said nucleotide sequence or by the polymerase chain reaction with primers also synthesized according to said information, if necessary.

To express the desired gene or generally speaking the desired DNA sequence of the present invention efficiently, various promoters can be used; for example, the original promoter of said gene, promoters of antibiotic resistance genes such as the kanamycin resistant gene of Tn5 (Berg, D. E., and C. M. Berg. 1983. Bio/Technology 1:417-435), the ampicillin resistant gene of pBR322, a promoter of the beta-galactosidase gene of *Escherichia coli* (lac), trp-, tac- trc-promoter, promoters of lambda phages and any promoters which can be functional in the hosts consisting of microorganisms including bacteria such as *E. coli, P. putida, Acetobacter xylinum, A. pasteurianus, A. aceti, A. hansenii* and *G. oxydans*, mammalian and plant cells.

Furthermore other regulatory elements, such as a Shine-Dalgarno (SD) sequence (for example, AGGAGG etc. including natural and synthetic sequences operable in the host cell) and a transcriptional terminator (inverted repeat structure including any natural and synthetic sequence operable in the host cell) which are operable in the host cell into which the coding sequence will be introduced can be used with the above described promoters.

For the expression of periplasmic polypeptides (AADHs) a signal peptide, which contains usually 15 to 50 amino acid residues totally hydrophobic, is indispensable. A DNA encoding a signal peptide can be selected from any natural or synthetic sequence operable in the host cell.

A wide variety of host/cloning vector combinations may be employed in cloning the double-stranded DNA. Cloning vector is generally a plasmid or phage which contains a replication origin, regulatory elemtents, a cloning site including a multi-cloning site and selection markers such as antibiotic resistance genes including resistance genes for ampicillin, tetracycline, kanamycin, streptomycin, gentamicin, spectinomycin etc.

Preferred vectors for the expression of the DNA sequences of the present invention in *E. coli* are selected from any vectors usually used in *E. coli,* such as pBR322 or its derivatives including pUC18 and pBluescript II, pACYC177 and pACYC184 (J. Bacteriol., 134:1141-1156, 1978) and their derivatives, and a vector derived from a broad host range plasmid such as RK2 and RSF1010. A preferred vector for the expression of the DNA sequences of the present invention in*Gluconobacter* including *G. oxydans* DSM No. 4025 and *P. putida* is selected from any vectors which can replicate in *Gluconobacter* and/or *P. putida*, as well as a preferred cloning organism such as in *E. coli.* The preferred vector is a broad-host-range vector such as a cosmid vector like pVK102 and its derivatives and RSF1010 and its derivatives, and a vector containing a replication origin functional in *Gluconobacter* and another origin functional in *E. coli.* Copy number and stability of the vector should be carefully considered for stable and efficient expression of the cloned gene and also for efficient cultivation of the host cell carrying the cloned gene. DNA molecules containing transposable elements such as Tn5 can be also used as a vector to introduce the DNA sequence of the present invention into the preferred host, especially on a chromosome. DNA molecules containing any DNAs isolated from the preferred host together with the desired DNA sequence of the present invention are also useful to introduce the desired DNA sequence of the present invention into the preferred host, especially on a chromosome. Such DNA molecules can be transferred to the preferred host by transformation, transduction, transconjugation or electroporation.

Useful hosts may include microorganisms, mammalian cells, and plant cells and the like. As preferable microorganisms, there may be mentioned bacteria such as *E. coli, P.* putida, *A. xylinum, A. pasteurianus, A. aceti, A. hansenii, G. oxydans*, and any Gram-negative bacteria which are capable of producing recombinant AADHs. Functional equivalents, subcultures, mutants and variants of said microorganism can be also used in the present invention. A preferred strain is *E. coli* K12 and its derivatives, *P. putida* or *G. oxydans* DSM No. 4025.

The functional AADH encoding DNA sequence of the present invention is ligated into a suitable vector containing a regulatory region such as a promoter and a ribosomal binding site operable in the host cell described above using well-known methods in the art to produce an expression plasmid Structures of such recombinant expression vectors are specifically shown in Fig. 1 and 10.

To construct a recombinant microorganism carrying a recombinant expression vector, various gene transfer methods including transformation, transduction, conjugal mating (Chapters 14 and 15, Methods for general and molecular bacteriology, Philipp Gerhardt et al. ed., American Society for Microbiology, (1994), and electroporation can be used. The method for constructing a recombinant organism may be selected from the methods well-known in the field of molecular biology. Usual transformation systems can be used for *E. coli, Pseudomonas* and *Acetobacter*. A transduction system can also be used for *E. coli.* Conjugal mating systems can be widely used in Gram-positive and Gram-negative bacteria including *E. coli, P. putida* and *G. oxydans*. A preferred conjugal mating method is described in WO89/06688. The conjugation can occur in liquid media or on a solid surface. The preferred recipient is selected from *E. coli, P.* putida and *G. oxydans* which can produce active AADHs with a suitable recombinant expression vector. The preferred recipient for 2KGA production is *G. oxydans* DSM No. 4025. To the recipient for conjugal mating, a selective marker is usually added; for example, resistance against nalidixic acid or rifampicin is usually selected.

The AADHs provided by the present invention catalyze the oxidation of alcohols and/or aldehydes, and are thus capable of producing aldehydes, ketones or carboxylic acids from the corresponding substrates. More particularly, the AADHs provided by the present invention can catalyze the oxidation of L-sorbose to 2KGA via L-sorbosone and/or the oxidation of D-sorbitol to L-sorbose. More particularly, the AADHs provided by the present invention contain enzymes encoded by a DNA molecule according to SEQ ID NO: 4 or a DNA sequence which hybridizes under stringent hybridization and stringent washing conditions with said DNA molecule, said DNA encoding a polypeptide having alcohol and/or aldehyde dehydrogenase activity, and Enzyme B, which have the amino acid sequence shown in SEQ ID NO 8.

Enzyme B genes, which have the nucleotide sequence shown in SEQ ID NO. 4 and encode the polypeptides having the amino acid sequence shown in SEQ ID NO. 8 or enzymes encoded by a DNA molecule according to SEQ ID NO: 4 or a DNA sequence which hybridizes under stringent hybridization and stringent washing conditions with said DNA molecule, said DNA encoding a polypeptide having alcohol and/or aldehyde dehydrogenase activity can be derived from *G. oxydans* strain DSM No. 4025.

The AADHs including enzymes encoded by a DNA molecule according to SEQ ID NO: 4 or a DNA sequence which hybridizes under stringent hybridization and stringent washing conditions with said DNA molecule, said DNA encoding a polypeptide having alcohol and/or aldehyde dehydrogenase activity, and enzyme B provided by the present invention can be prepared independently by cultivating an appropriate organism, disrupting the cells and isolating and purifying them from cell free extracts of disrupted cells, preferably from the soluble fraction of the microorganism.

The recombinant host cells provided in the present invention may be cultured in an aqueous medium supplemented with appropriate nutrients under aerobic conditions. The cultivation may be conducted at a pH between about 4.0 and 9.0, preferably between about 6.0 and 8.0. While the cultivation period varies depending upon pH, temperature and nutrient medium used, usually 2 to 5 days will bring about favorable results. A preferred temperature range for carrying out the cultivation is from about 13°C to 45°C preferably from about 18°C to 42°C.

It is usually required that the culture medium contains such nutrients as assimilable carbon sources, digestible nitrogen sources and inorganic substances, vitamins, trace elements and other growth promoting factors. As assimilable carbon sources, glycerol, D-glucose, D-mannitol, D-fructose, D-arabitol, D-sorbitol, L-sorbose, and the like can be used.

Various organic or inorganic substances may also be used as nitrogen sources, such as yeast extract, meat extract, peptone, casein, corn steep liquor, urea, amino acids, nitrates, ammonium salts and the like. As inorganic substances, magnesium sulfate, potassium phosphate, ferrous and ferric chlorides, calcium carbonate and the like may be used.

In the following, the properties of the purified recombinant AADH enzymes specifically from *P. putida* and the production method are summerized.

(1) Enzyme activity

The AADHs of the present invention catalyze oxidation of alcohols and aldehydes including D-sorbitol, L-sorbose, and L-sorbosone in the presence of an electron acceptor according to the following reaction formula.

Alcohol+ Electron acceptor -> Aldehyde + Reduced electron acceptor

Alcohol+ Electron acceptor -> Ketone + Reduced electron acceptor

Aldehyde + Electron acceptor -> Carboxylic acid + Reduced acceptor

Sugar alcohol + Electron acceptor -> Aldose + Reduced electron acceptor

Sugar alcohol + Electron acceptor -> Ketose + Reduced electron acceptor

Aldehyde ketose+Electron acceptor -> Ketocarboxylic acid+Reduced electron acceptor

Carboxylic acid+Electron acceptor -> Ketocarboxylic acid+Reduced electron acceptor

The enzymes do not utilize molecular oxygen as an acceptor. As an acceptor, 2,6-dichlorophenolindophenol (DCIP), phenazine methosulphate (PMS), Wurster's blue, ferricyanide, coenzyme Q or cytochrome c can be used.

**[0043]** - One unit of enzyme activity was defined as the amount of enzyme which catalyzed the reduction of 1 μmole of DCIP per minute. The extinction coefficient of DCIP at pH 8.0 was taken as 15 $mM^{-1}$. The standard reaction mixture (1.0 ml) contained 0.1 mM DCIP, 1 mM PMS, 2 to 125 mM substrate, 50 mM Tris-malate-NaOH buffer (pH 8.0), and 10 μl of the enzyme solution. A reference cuvette contained all the above components except the substrate.

(2) Properties of the AADHs

a) Substrate specificity and products of the enzymatic reaction

The Enzyme B was characterized by its substrate specificity as described above using 8 substrates: n-propanol, isopropanol, D-glucose, D-sorbitol, L-sorbosone, D-mannitol, L-sorbose, and D-fructose. The results are indicated in Table 1.

Table 1. Substrate specificity of the Enzyme B (units/mg of purified protein)

| Substrate | Enzyme B |
|---|---|
| 50 mM n-Propanol | 40.0 |
| 50 mM Isopropanol | 72.3 |
| 50 mM D-Glucose | 943.9 |
| 125 mM D-Sorbitol | 130.9 |
| 2mM L-Sorbosone | 73.6 |
| 50 mM D-Mannitol | 517.4 |
| 125 mM L-Sorbose | 8.4 |
| 125 mM D-Fructose | 2.1 |

Enzyme B showed a high reactivity for D-glucose or D-mannitol, but relatively low reactivity for n-propanol and isopropanol.

Product(s) formed from a substrate in the reaction with Enzyme B was analyzed by thin layer chromatography (TLC) and/or high performance liquid chromatography (HPLC) with authentic compounds. Enzyme B (designated B group) converted D-glucose, D-sorbitol, L-sorbosone, D-mannitol, L-idose, glycerol, D-gluconic acid, D-mannoic acid to D-gluconate, L-sorbose, 2KGA, D-fructose, L-idonic acid, dihydroxyacetone, 5-keto-D-gluconic acid, and 5-keto-D-mannoic acid, respectively. In the analogy to the reactivity for L-sorbosone, D-glucosone can be converted to 2KD by the above mentioned AADH. Enzyme B showed the activity for alcohols including sugar alcohol such as D-sorbitol and D-mannitol, and aldehydes including aldose such as D-glucose and ketose such as L-sorbosone.

b) Optimum pH

Enzyme B has its optimal point at pH 8.0 - 8.5 as shown in Table 2.

Enzyme B has a relatively wide pH range toward a lower pH .

Table 2. Optimal pH of Enzyme B
(Relative activity, %)

| pH | Enzyme B |
|---|---|
| 6.0 | 21.0 |
| 6.5 | 51.6 |
| 7.0 | 61.6 |
| 7.5 | 75.3 |
| 8.0 | 100.0 |
| 8.5 | 62.2 |
| 9.0 | 8.0 |
| 9.5 | 0.0 |

c) pH stability

Enzyme B was incubated in buffers of various pH-values for 3 hours at 25 °C and the residual activitie was assayed and expressed as relative values against that obtained by no incubation at pH8. Enzyme B was stable between pH 6 to 9 as shown in Table 3.

Table 3. pH stability
(Relative activity, %)

| pH | Enzyme B |
|---|---|
| 4.0 | 25.2 |
| 5.0 | 56.1 |
| 6.0 | 100.9 |
| 7.0 | 101.9 |
| 8.0 | 114.0 |
| 9.0 | 101.9 |
| 10.0 | 85.5 |
| 11.0 | 70.1 |

d) Thermal stability

The residual activities after the treatment of enzyme B at 4, 20, 30, 40, 50, and 60°C for 5 minutes is shown in Table 4.

Table 4. Thermal stability of the enzymes
(Relative activity, %)

| Temperature | Enzyme B |
|---|---|
| 4 °C | 100.0 |
| 20 °C | 97.2 |
| 30 °C | 95.4 |
| 40 °C | 84.6 |
| 50 °C | 29.2 |
| 60°C | 0.0 |

e) Effect of metal ions and inhibitors

Remaining activities after the treatment of enzyme B with various metals and inhibitors are shown in Table 5. $MgCl_2$ and $CaCl_2$ were nearly inert to enzyme B while the other metal ions, especially $CuCl_2$, significantly affected the reactivity. Enzyme B was less inhibited by EDTA and EGTA.

Table 5. Effect of metals and inhibitors on enzyme B activity
(Relative remaining activity)

| Compound | Enzyme B |
|---|---|
| | Substrate D-Sorbitol |
| 5mM $CoCl_2$ | 23.6 |
| 5mM $CuCl_2$ | 0.0 |
| 5mM $ZnCl_2$ | 0.0 |
| 5mM $MgCl_2$ | 100.0 |
| 5mM $CaCl_2$ | 102.9 |
| 5mM $MnCl_2$ | 0.0 |
| 5mM $FeCl_2$ | 5.9 |
| 5mM $FeCl_3$ | 0.0 |
| 5mM $NiSO_4$ | 79.4 |
| 10mM EDTA | 91.3 |
| 10mM EGTA | 74.0 |
| 1mM NaF | 100.8 |
| 2mM NEM | 100.8 |
| 1mM $ICH_2COONa$ | 100.2 |
| 0.5mM Hydroxylamine-HCl | 102.1 |

f) Molecular weight and subunit

Enzyme B purified from *P. putida* transconjugants consists of one type of unit with the molecular weight of about 60,000, as measured by sodium dodecyl sulfate polyacrylamide gel electrophoresis.

g) N-terminal amino acid sequence

N-terminal sequences of mature Enzyme B is
Enzyme B : Gln-Val-Thr-Pro-Ile-Thr-Asp-Glu-Leu-Leu-Ala----.

(3) Production of the AADHs

Cells are harvested from the fermentation broth by centrifugation or filtration. The cells are suspended in the buffer solution and disrupted by means of a homogenizer, sonicator or treatment with lysozyme and the like to give a disrupted solution of cells.

AADHs are isolated and purified from a cell free extract of disrupted cells, preferably from the soluble fraction of the microorganisms by usual protein purification methods such as ammonium sulfate precipitation, dialysis, ion exchange chromatographies, gel filtration chromatographies, and affinity chromatographies.

(4) Enzyme reaction

Enzyme reaction was performed at pH values from about 6.0 to about 9.0 at the temperature of about 10°C to about 50°C, preferrably of 20°C to 40°C in the presence of an electron acceptor, for example, DCIP, PMS, Wurster's blue, ferricyanide, coenzyme Q, cytochrome c and the like in a buffer such as Tris-HCl buffer, phosphate buffer and the like. The concentration of the substrate in a reaction mixture can vary depending on the other reaction conditions but, in general, is desirable to be about 1 - 200 g/l, most preferably from 1 - 100 g/l.

In the enzyme reaction, AADHs may also be used in an immobilized state with an appropriate carrier. Any means of immobilizing enzymes generally known to the art may be used. For instance, the enzyme may be bound directly to membrane granules or the like of a resin having functional group(s), or it may be bound through bridging compounds having functional group(s), for example, glutaraldehyde, to the resin.

The enzymatic polypeptides of the present invention are usually produced in the form of dimers. Such dimers contain homodimers of Enzyme B or of enzymes encoded by a DNA molecule according to SEQ ID NO: 4 or a DNA sequence which hybridizes under stringent hybridization and stringent washing conditions with said DNA molecule, said DNA encoding a polypeptide having alcohol and/or aldehyde dehydrogenase activity, and heterodimers consisting of two different enzymatic polypeptides mentioned above. Thus the recombinant enzyme preparation of the present invention also contain one or more of said homodimers and/or heterodimers.

The recombinant organisms provided by the present invention are highly useful for the production of the recombinant enzyme preparations of AADHs having an alcohol and/or aldehyde dehydrogenase activity. Said organisms are also useful for the production of aldehydes, carboxylic acids and ketones, especially, 2KGA by utilizing said recombinant enzyme preparations, and by utilizing said recombinant organisms.

The production of 2KGA with the said recombinant organisms can be performed in the fermentation with the medium and culture conditions as described above. The production of 2KGA may be performed with the recombinant organisms described above together with the concomitant organisms such as *E. coli, P. putida* and *Bacillus megaterium*.

## Examples

### Example 1. Cloning of AADH genes

(1) Construction of a genomic library of *G. oxydans* DSM No. 4025

Chromosomal DNA was prepared as follows. *G. oxydans* DSM No. 4025 was cultivated on an agar plate containing 20 ml of NS2 medium consisting of 5.0% D-mannitol, 0.25% $MgSO_4 \cdot 7H_2O$, 1.75% corn steep liquor, 5.0% baker's yeast (Oriental Yeast Co., Osaka, Japan), 0.5% $CaCO_3$, 0.5% urea (sterilized separately) and 2.0% agar (pH 7.0 before sterilization) at 27°C for 3 days. The cells were collected from the agar plate, washed with 10 ml of 10 mM Tris-HCl buffer (pH 8.0) containing 1mM EDTA and resuspended in 5 ml of 10 mM Tris-HCl buffer (pH 8.0) containing 20 mM EDTA. The cell suspension was treated with lysozyme (Sigma Chemicals Co., St. Louis, Mo., USA) at a final concentration of 400 μg/ml at 37°C for 30 minutes, then with pronase (400 units) at 37°C 30 minutes and with 1% SDS at 37°C for 1 hour. Chromosomal DNA was treated with phenol and RNase A (Boheringer Mannheim, GmbH, Mannheim, Germany) according to the method described by Maniatis et al. (Molecular cloning: a laboratory manual. Cold Spring Harbor Laboratory, Cold Spring Harbor, N. Y., (1982). Chromosomal DNA (200 μg) was digested with 168 units of *Sal*I (Boehringer Mannheim) at 37°C for 5 to 90 minutes. The resulting partially digested fragments of 15 - 35 kb were isolated by preparative agarose gel electrophoresis (agarose: 0.7%); the gel piece containing the desired fragments was cut out and the DNAs were electro-eluted from the gel into TAE buffer consisting of 40 mM Tris-acetate and 2 mM EDTA. Thus, 40 μg of the DNAs were obtained. In parallel, 8 μg of the cosmid vector pVK102 (ATCC 37158) was completely digested with *Sal*I and treated with calf intestine alkaline phosphatase (Boehringer Mannheim) according to the supplier's recommendation. pVK102 (0.4 μg) was ligated with the 15-35 kb *Sal*I fragments (0.2 - 2 μg) by the ligation kit (Takara Shuzo Co. Ltd., Kyoto, Japan) at 26°C for 10 minutes. The ligated DNAs were then used for in vitro packaging according to the method described by the supplier (Amersham): mixing the ligated DNAs with the phage coat protein parts. The resulting phage particles were used to infect *E. coli* ED8767 (Murray, N. E., W. J. Brammar and K. Murray. Mol. Gen. Genet., 150:53-61, 1977). About 3,000 $Km^r$ $Tc^S$ colonies were obtained and all of the colonies tested (24 colonies) possessed the insert DNAs; its average size was 26.5 kb. Another cosmid library of *G. oxydans* DSM No. 4025 containing 55,000 clones was constructed by using chromosomal DNA of *G. oxydans* DSM No. 4025 partially digested with *Eco*RI and inserting them into theEcoRI site of pVK100 by almost the same method described above. All of the colonies tested (24 colonies) possessed insert DNAs (average size; 27 kb).

These two cosmid libraries in *E. coli* ED8767 were then transferred into *E. coli* S 17-1 (Tra+, Bio/Technology, 1:784-791, 1983) by using the mixture of recombinant plasmid DNAs extracted from *E. coli* ED8767 libraries. About 4,000 $Km^r$ transformants of *E. coli* S17-1 were picked up, cultivated individually in microtiter plates containing 100 µl of LB consisting of 10 g/l of Bactotrypton (Difco), 5 g/l of yeast extract (Difco) and 5 g/l of NaCl supplemented with 50 µg/ml kanamycin at 37°C, and stocked with 15% glycerol at -80°C as cosmid libraries in *E. coli* S17-1.

The *G. oxydans* DSM No. 4025-*Sal*I and -*Eco*RI cosmid libraries were constructed in *E. coli* S17-1. From the library, 1,400 clones were individually transferred from *E. coli* S17-1 into *P. putida* ATCC 21812 by conjugal mating. 1,400 cultures stocked in microtiter plates at -80°C were thawed and transferred to microtiter plates containing 100 µl of fresh LB medium in each well with a plate transfer cartridge (Nunc) and cultivated at 37°C overnight. Nalidixic acid resistant ($Nal^r$) *P. putida* ATCC21812 was cultivated at 30°C overnight in 100 ml of MB medium consisting of 2.5% mannitol, 0.5% yeast extract (Difco Laboratories, Detroit, Mich.) and 0.3% Bactotryptone (Difco). Fifty µl of the *P. putida* culture was individually added to the 1,400 wells containing cultures of the cosmid library. The 1,400 cell mixtures were spotted with plate transfer cartridges onto nitrocellulose filters placed on the surface of FB agar medium consisting of 5% fructose, 1% yeast extract (Difco), 1% polypeptone (Daigo Eiyo, Japan) and 1.8% agar and cultivated at 27°C overnight. Nalidixic acid was used for the counter-selection of transconjugants against donor *E. coli*. The cells grown on the filters were individually streaked onto MB agar medium containing 50 µg/ml of nalidixic acid and 50 µg/ml of kanamycin hereinafter referred to as (MNK agar plate) and incubated for 4 days at 27°C for the selection of transconjugants. The resulting colonies were purified by streaking on MNK agar plates as mentioned above. Thus, 1,400 transconjugants of *P. putida* [gene library of *G. oxydans* DSM No. 4025 in *P. putida*] were prepared.

(2) Immunological screening of clones of the AADH gene of *G. oxydans* DSM No. 4025.

At first, 350 transconjugants (175 from *Sal*I library and 175 from *Eco*RI library) maintained MNK agar plates were individually cultivated in test tubes containing 5 ml of MNK medium. The cells were collected from 1.5 ml of each broth and treated for Western-blot analysis as follows. The cells were suspended in 50 µl of Laemmli buffer consisting of 62.5 mM Tris-HCl, pH 6.8, 10% glycerol, 5% mercaptoethanol and 2% SDS. The cell suspension was boiled for 3 minutes, and 10 µl of the cell lysate was applied on SDS-PAGE. The resulting protein bands were then electro-blotted to a nitrocellulose filter by an electroblotting apparatus (Marysol Industrial Co., Ltd.) operated at 40 V, 200 mA for 16 hours in 2.5 mM Tris-19.2 mM glycine buffer, pH 8.6, containing 20 % methanol. The filter was, then, incubated for 1 hour in 3% gelatin in TBS buffer consisting of 20 mM Tris, pH 7.5, and 500 mM NaCl. After a brief rinse in TTBS buffer consisting of 20 mM Tris, pH 7.5, 500 mM NaCl and 0.05% Tween 20, the filter was incubated for 1 hour with a first-antibody which contained 1:500-diluted anti-AADH antibody in TTBS buffer containing 1% gelatin. The anti-AADH antibody had been prepared by mixing the AADH proteins purified from *G. oxydans* DSM No. 4025 with incomplete adjuvant, injecting the resulting mixture into a white rabbit twice with 2 weeks' interval, collecting whole blood 1 week after the second injection and preparing the serum fraction as the anti-AADH antibody. Then, the filter was washed twice (5 min each) in TTBS buffer and incubated for 1 hour in a second-antibody (goat anti-rabbit IgG-horseradish peroxidase conjugate) solution which contained 1:3,000-diluted second antibody in TTBS containing 1% gelatin. After washing in TTBS buffer twice and in TBS once, the filter was immersed in a color developing solution until blue bands became visible with Konica Immunostaining HRP Kit IS-50B (Konica, Tokyo, Japan) according to the supplier's recommendation. For an actual screening, five cell lysates were mixed and applied to one well for the first Western-blot screening. Out of 70 mixtures, 14 exhibited positive bands; nine samples had immuno-reactive proteins of approximate Mr 64,000, but two of these exhibited weak signals; one had an immuno-reactive protein of approximate Mr 60,000; and four samples had immuno-reactive proteins of Mr 55,000.

Seven mixture samples showing strong signals at Mr 64,000 were individually subjected to a second Western-blot screening to identify the clone in each mixture. One positive clone per one mixture samples was identified; plasmids of the seven clones were designated as p6E10, p16C8, p16F4, p17E8, p1E2, p24D4, and p26C3, respectively. By restriction enzyme analysis, it was found that four plasmids, p6E10, p16C8, p16F4, and p17E8, carried the same DNA region and the other three carried different regions from that of the former four plasmids.

(3) Screening of the AADH genes from the cosmid libraries by colony-blot and Southern-blot hybridization

To find the other AADH genes besides the genes obtained by the immunological screening as described above, the whole cosmid libraries of *G. oxydans* DSM No. 4025 in *E. coli* ED 8767 (*Sal*I-library and *Eco*RI-libraries) were screened by colony- and Southern-blot hybridization with a 0.9 kb *Sal*I fragment of p24D4. The 0.9 kb *Sal*I fragment hybridized with a oligonucleotide probe, ATGATGGT(GATC)AC(GATC)AA(TC)GT synthesized according to an internal amino acid sequence of the natural AADH enzyme purified from *G. oxydans* DSM No. 4025, MetMetValThrAsnValAspVal-GlnMetSerThrGlu, which was obtained by digestion and sequenced by automatic gas-phase sequencer (Applied Biosystems 470A). The cells of the cosmid libraries were appropriately diluted and spread on LK agar plates, and the resulting colonies were blotted onto nylon filters and were analyzed by hybridization with the $^{32}P$-labeled 0.9 kb *Sal*I fragment. About 1% of the colonies showed positive signals; 41 colonies were selected from the *Sal*I library and 20 from *Eco*RI library, and they were subjected to restriction enzyme analysis, followed by Southern-blot analysis. Six different AADH gene-related DNA regions were isolated in this screening as follows: four already-isolated regions carried on p24D4, p1E2, p26C3 and, p17E8, and two new regions carried on two separate plasmids designated as pSS31 and pSS53. The other plasmid pSS33 carried both of the two regions which were carried on p24D4 and pSS31.

(4) Immunological and enzymatic characterization of AADH clones

Western-blot analysis of cell lysates of *P. putida* carrying p24D4, p1E2, p26C3, pSS31 and p17E8 showed that the five clones encoded proteins with molecular weights of about 64,000, 62,500, 62,500, 60,000 and 62,000, respectively. Plasmid pSS33 encoded two immuno-reactive proteins with molecular weights of about 64,000 and 60,000, whereas pSS53 did not produce any immuno-reactive proteins.

Enzyme activities of each clone (cell free extract, soluble fraction and membrane fraction) were measured by photometric analysis. The cells of each clone were inoculated in 5 ml of MB medium in a test tube and cultivated at 30°C for 24 hours. The resulting broth was transferred into 200ml of fresh MB medium in 500 ml flask and the flask was shaken on the rotary flask shaker at 30°C for 24 hours. The cells were collected by centrifugation at 6,000 x g for 10 minutes and washed with 40 ml of cold buffer consisting of 50 mM Tris-HCl, pH 7.5, 5mM $MgCl_2$ and 0.5 mM phenylmethylsulfonyl fluoride and suspended with the same buffer to prepare cell suspension of 1 g wet cells per 5ml. The cell suspension was subjected twice to a French press cell disruptor (1,500 $kg/cm^2$) and the resulting homogenate was centrifuged at 6,000 x g for 10 minutes to remove cell debris. Thus obtained cell free extract (CFE) was centrifuged at 100,000 x g for 60 minutes. The resulting supernatant and pellet were collected as the cytosol fraction and the membrane fraction, respectively and subjected to PMS-DCIP assay as follows. The enzyme reaction mixture (1.0 ml) contained 100 μM DCIP, 1mM PMS, 50 mM Tris malate-NaOH buffer, pH 8.0, a substrate and the enzyme (10 μl). Substrate-dependent decreasing rate of absorbance of DCIP at 600 nm was measured at 25°C by using a Kontron spectrophotometer UVIKON 810. Table 6 shows the level of enzyme activities in the cell free extract and the soluble fractions of the clones. According to the substrate specificity, the enzyme encoded on each plasmid was classified into large three groups, A-, B- and C-groups: A-group catalyzes the oxidation of L-sorbose, D-sorbitol and 1-propanol; B-group catalyzes the oxidation of D-glucose and D-sorbitol; C-group showed no clearly detectable activities on the substrates used. In the A-group, there were three types, A, A' and A" each of which was distinguished from each other by their physical map of the DNA carried on each plasmid. B- or C-group each consisted of only one type of protein derived from one region of the chromosomal DNA.

Table 6.

| Enzyme Group | Enzyme Name | Plasmid | CFE | Soluble fraction | | | | |
|---|---|---|---|---|---|---|---|---|
| | | | Sorbose 125 mM | Sorbose*1 125 mM | Glucose*2 50 mM | Sorbitol*3 125 mM | Sorbosone*4 2 mM | n-Propanol 50 mM |
| A | A | p24D4 | +++ | | - | +++ | +++ ++++ | |
| A | A' | p1E2 | + | + | - | + | + | + |
| A | A" | p26C3 | + | + | - | +/- | + | + |
| B | B | pSS31 | - | - | ++++ | ++ | + | + |
| C | - | p17E8 | | - | +/- | - | - | - |

(continued)

| Enzyme Group | Enzyme Name | Plasmid | CFE | Soluble fraction | | | | |
|---|---|---|---|---|---|---|---|---|
| | | | Sorbose 125 mM | Sorbose*1 125 mM | Glucose*2 50 mM | Sorbitol*3 125 mM | Sorbosone*4 2 mM | n-Propanol 50 mM |
| A and B | A and B | pSS33 | +++ | +++ | ++++ | ++++ | +++ | ++++ |

Level of the activity; ++++ : very high
+++ : high
++ : medium
+ : low
+/- : trace
not detected
*1 - *4:Oxidation product of each substrate was determined by a resting cell reaction followed by TLC analysis.
*1: Oxidation product of L-sorbose by Enzymes A, A', A", and [A and B] was 2KGA.
*2: Oxidation product of D-glucose by Enzyme B, and Enzyems [A and B] was D-gluconic acid.
*3: Oxidation product of D-sorbitol by Enzymes A, A', and A" was mainly D- glucose; that by Enzyme B was L-sorbose; and that by Enzymes [A and B] was mixture of D-glucose and L-sorbose.
*4: Oxidation product of L-sorbosone by Enzymes A, A', A", B, and [A and B] was 2KGA.

Example 2. Nucleotide sequencing

Nucleotide sequences of the genes for Enzymes A, A', A" and B were determined with the plasmids, p24D4, p1E2, p26C3, and pSS31, respectively, by the dideoxynucleotide chain termination method using M13mp18 and M13mp19 (Boehringer Mannheim). One open reading frame (ORF) for each gene was found; the nucleotide sequences of the four genes are shown in the sequence list SEQ ID No. 1 to 4 and the amino acid sequences deduced from the nucleotide sequences were shown in the sequence list SEQ ID No. 5 to 8. The ORF B gene is 1737-bp long and encodes 579 amino acid residues all including 23 amino acid of signal sequences.

The homologies between Enzymes A, A', A" and B are shown in Table 7.

Table 7. Homologies of amino acid sequences among AADHs.

| | (%) | | | |
|---|---|---|---|---|
| | Enzyme A | Enzyme A' | Enzyme A" | Enzyme B |
| Enzyme A | 100 | - | - | - |
| Enzyme A' | 89 | 100 | - | - |
| Enzyme A" | 85 | 86 | 100 | - |
| Enzyme B | 83 | 82 | 81 | 100 |

Figure 5 shows the amino acid sequences of mature Enzyme A and Enzyme B which are aligned so as to be comparable.

Homology search of Enzymes A, A', A" and B revealed that Enzymes A, A', A" and B showed rather low homology (26 - 31 % homology through the polypeptides) with several quino-proteins including alcohol dehydrogenase of *Acetobacter aceti* (T. Inoue et al., J. Bacteriol. 171: 3115-3122) or *Acetobacter polyoxogenes* (T. Tamaki et al., B. B. A., 1088:292-300), and methanol dehydrogenase of *Paracoccus denitrificans* (N. Harms et al., J. Bacteriol., 169: 3966-3975), *Methylobacterium organophilum* (S. M. Machlin et al., J. Bacteriol., 170: 4739-4747), or *Methylobacterium extorquens* (D. J. Anderson et al., Gene 90: 171-176).

Example 3. Subcloning of AADH genes

Enzyme B gene was originally cloned as a cosmid clone of pSS31 which has about 30kb insert in *Sal*I site of pVK102. It was subcloned as 6.5kb *Bgl*II fragment into *Bgl*II site of pVK101 (ATCC 37157) to obtain pSSB102. Then, it was further subcloned to use as a Enzyme B gene cassette. The 6.5 kb *Bgl*II fragment was cloned into *Bam*HI site of pUC18 to obtain pSSB202. Then, 2.3 kb *Xho*II fragment was excised from pSSB202. The 2.3 kb *Xho*II fragment includes ORF of Enzyme B with 120 bp of 5'-noncoding region and about 500 bp of 3'-noncoding region. The fragment was treated with Klenow fragment to fill-in the cohesive ends and cloned into *Hind*III site of pUC18 with *Hind*III linker to produce pSSB203. The Enzyme B gene cassette (2.3 kb *Hind*III fragment) was inserted at *Nind*III site of pVK102 to make pSSB103R. The plasmid pSSB103R was introduced into nalidixic acid resistant *P. putida* [ATCC 21812] by a conjugal mating method, and the transconjugant of *P. putida* carrying pSSB103R was selected on MNT agar medium and subjected to a mini-resting cell reaction. *P. putida* carrying pSSB103R showed the Enzyme B activity (L-sorbose formation from D-sorbitol) in the resting cell reaction. (Incidentally, XhoII fragment was found not to be a *Xho*II-*Xho*II fragment, but a *Xho*II-*Xho*I fragment as a result of nucleotide sequencing. *Xho*I might be present in the *Xho*II preparation.)

Example 4. Isolation and characterization of AADHs from transconjugants of *P. putida*.

(1) Cultivation of microorganisms

*P. putida* [ATCC 21812] carrying cosmid vector pVK102 containing the Enzyme B gene; pSSB103R was cultivated in MB broth in the presence of antibiotic. Antibiotics added into medium was as follows; 5 μg/ml tetracycline for pSSB103R (Enzyme B). From the agar plate of MB containing the respective antibiotic, the cells were inoculated in 10 test tubes containing 5 ml MB medium with the respective antibiotic and cultivated with shaking at 30°C. After 2 days of cultivation, the cells were transferred to ten 500 ml-Erlenmeyer flasks containing 100 ml of the same medium and cultivated with shaking at 30°C. After 1 day of cultivation, the seed cultures were combined and transferred to 18 liters of the medium in 30 L jar fermenter (Marubishi) and cultivated for 18 hours with 300 rpm agitation and 1.0 vvm aeration at 30°C. The cells were harvested by centrifuge at 6,000 x g for 10 minutes, washed once with 1.5 liters of 25 mM Tris-HCl, pH 7.5, containing 5 mM $CaCl_2$, 1 mM $MgCl_2$, 0.2 M NaCl, 2.5% sucrose, and 0.5 mM PMSF and stocked at - 20°C until use. As a result, about 150 g wet weight cells were obtained.

(2) Purification of the cloned Enzyme B.

Purification of the Enzyme B was carried out by the same procedure with almost the same scale. All operations were carried out at 4 - 10°C unless otherwise stated. The enzyme activity determination for Enzyme B was carried out with the substrates, L-sorbose, n-propanol, n-propanol and D-glucose, respectively, by spectrophotometric assay as described in Example 1 throughout the purification steps. The cells (about 100 g wet weight cells containing 8 - 10 g of total proteins) were thawed and suspended in about 200 ml of 25 mM Tris-HCl, pH 8.0, and disrupted by passing through French press (1500 kg/$cm^2$) twice. Then, DNase and $MgCl_2$ were added to the suspension at the final concentration of 0.01 mg/ml and 1 mM, respectively, to reduce viscosity of the solution due to DNA. Cell debris was removed by centrifugation at 6,000 x g for 10 minutes. The suspension was filled up to 240 ml with the 25 mM Tris-HCl buffer, pH 8.0, and centrifuged at 100,000 x g for 90 minutes to remove insoluble membrane fraction. The soluble supernatant was filled up to 240 ml with the Tris buffer and, then, pyrroloquinoline quinone (PQQ) and $CaCl_2$ were added at the final concentration of 12.5 μM and 5 mM, respectively, and the solution was stirred vigorously for 15 minutes at room temperature. The soluble fraction prepared as above was fractionated by $(NH_4)_2SO_4$. The fraction 35 - 60%-saturated $(NH_4)_2SO_4$ was precipitated and resuspended in 100 ml of 25 mM Tris-HCl buffer, pH 8.0, containing 5 mM $CaCl_2$, and 5% sucrose and, then, PQQ was added again at the final concentration of 12.5 μM. The enzyme solution was dialyzed against 1000 ml of the same buffer (without PQQ) overnight. Twenty grams of solid polyethylene glycol #6000 was added to the dialysate slowly with gentle stirring. After stirring for 30 minutes, precipitates were removed by centrifugation at 10,000 x g for 20 minutes, and the supernatant was filled up to 200 ml with the buffer indicated as above.

The enzyme solution prepared as above was purified by following three chromatography steps.

The first step: DEAE-Toyopearl 650M

The crude enzyme solution was subjected to a column of DEAE-Toyopearl 650M (2.5x 40 cm) which had been equilibrated with 25 mM Tris-HCl buffer, pH 8.0, containing 5 mM $CaCl_2$, and 5% sucrose. The column was washed with 400 ml of the same buffer and the enzyme was eluted by 2,000 ml of 0 - 0.5 M NaCl linear gradient in the buffer at a flow rate of 150 ml/hour. The enzyme active fractions were pooled and diluted 2-fold with the buffer without NaCl.

The second step: Q-Sepharose (Fast Flow)

The enzyme solution was subjected to a column of Q-Sepharose (Fast Flow) (1.5 x 20 cm) which had been equilibrated with the buffer without NaCl. The column was washed with 200 ml of the buffer containing 0.2 M NaCl and the enzyme was eluted by 600 ml of 0.2 - 0.6 M NaCl linear gradient in the buffer at a flow rate of 50 ml/hour. The enzyme active fractions were pooled and concentrated to 2.5 ml by using ultrafilter:Amicon, PM-30 under $N_2$ gas.

The third step: Sephacryl S-300 HR (gel filtration)

The concentrated enzyme was filtrated by a column of Sephacryl S-300 HR (2.5 x 100 cm) which had been equilibrated with 25 mM HEPES, pH 7.5, containing 5 mM $CaCl_2$, 5% sucrose, and 0.2 M NaCl. The column was developed by the same buffer at a flow rate of 20 ml/hour. The enzyme active fractions were pooled and concentrated to below 1 ml by the ultrafilter mentioned above and, then, stocked at -80°C. The enzymes concentrated in the HEPES buffer was stable for at least 2 months at -80°C.

Consequently, 5.0 mg of Enzyme B were obtained.

(3) Properties of the Enzyme B.

a) Molecular weight and subunit.

The Enzyme B was eluted at the same position from the same gel filtration column on Sephacryl S-300HR under the same condition. The molecular weight of the enzymes was estimated as approximately 135,000 comparing with the molecular weight standard proteins (SDS-PAGE Standards, Low Range, Bio-Rad Laboratories, Richmond, CA, USA). The Enzyme B showed homogeneous single band on SDS-PAGE analysis with molecular weight of 60,000. Enzyme band B was detected on Western blotting analysis using anti-AADH rabbit serum. Therefore, it was concluded that the enzymes consisted of two identical subunits as an homo-dimeric form.

b) N-terminal amino acid sequence and amino acid composition.

N-terminal amino acid sequence of the mature Enzyme B was analyzed with automatic gas-phase sequencer (470A; Applied Biosystems) by Edman method [Acta Chem. Scand., 4, 283-293, {1950)]. The results were as follows:

Enzyme B : Gln-Val-Thr-Pro-Ile-Thr-Asp-Glu-Leu-Ala-.

The determined sequence of Enzyme B was identical to the sequence (starting from the twenty-fourth residues) deduced from the nucleotide sequence described in SEQ ID No. 8; these results indicate that the initial 23 residues of the enzyme are the signal sequences.

c) Substrate specificity

The Enzyme B was characterized by its substrate specificity on PMS-DCIP assay as described above using 8 substrates, n-propanol, isopropanol, D-glucose, D-sorbitol, L-sorbosone, D-mannitol, L-sorbose, and D-fructose. The results were indicated in Table 1.

d) Physicochemical property

Physicochemical studies of optimal pH, pH stability and thermal-stability, of the Enzymes B (as D-sorbitol dehydrogenase activity), was performed by the PMS-DCIP assay.

Table 2 summarizes the results of optimal pH of the enzyme. The enzyme activity was assayed by the PMS-DCIP spectrophotometric assay using various pH buffers. The buffers were 50 mM Tris-malate-NaOH, pH 6.0, 6.5, 7.0, 7.5, 8.0 and 8.5; 50 mM glycine-NaOH, pH 9.0 and 9.5. The extinction coefficients of DCIP at pH 6.0, 6.5, 7.0, 7.5, 8.0, 8.5, 9.0 and 9.5 were taken as 10.8, 13.2, 14.5, 14.9, 15.0, 15.1, 15.1 and 15.1, respectively. Enzyme B showed optimal points at pH 8.0 - 8.5. The Enzyme B had relatively wide pH range toward lower pH .

Table 3 indicates the results of pH-stabilities of the enzyme. The enzyme (about 0.01 mg/ml) was incubated with 50 mM buffer containing 5% sucrose, 0.2 M NaCl, and 5mM $CaCl_2$ at 25°C for 3 hours and assayed by PMS-DCIP spectrophotometric method. The buffers were Na-acetate, pH4 and 5, Tris-malate-NaOH, pH 6, 7 and 8, glycine-NaOH, pH 9 and 10. The values in the table are expressed as relative activity against that obtained by no incubation at pH 8.0. The substrates used was 125 mM D-sorbitol for Enzyme B. Profile of pH-stability of Enzyme B was stable at the range of pH 6 to 9.

Table 4 indicates the results of thermal-stabilities of the enzyme. The enzyme (about 0.05 mg/ml) in 25 mM HEPES buffer, pH 7.5, containing 5% sucrose, 0.2M NaCl, and 5 mM $CaCl_2$ was incubated at temperature indicated in the table (4 - 60°C) for 5 minutes, cooled in ice bath and assayed by PMS-DCIP spectrophotometric method. Remaining activity was expressed as relative activity against that obtained by 4°C incubation. The substrates used was 125 mM D-sorbitol for Enzyme B. After the treatment of the enzyme at 40°C for 5 min, the residual activity of Enzyme B was 70 - 85%.

e) General inhibitors

The enzyme (about 0.05 mg/ml) in 25 mM HEPES buffer, pH 7.5, containing 5% sucrose was incubated with metal or inhibitor for 30 minutes at 25°C. Remaining activity was assayed by PMS-DCIP spectrophotometric assay as described in Example 1. Remaining activity is expressed as relative activity against blank incubation. Effects of metal ions on the enzymes are listed in Table 5. $MgCl_2$ and $CaCl_2$ were nearly inert to the enzymes, while the other metal ions, especially $CuCl_2$, significantly affected. Effects of inhibitors on the enzyme are also included in Table 5. Enzyme B was less inhibited by EDTA and EGTA.

Example 5. Efficient production of Enzyme B in *E. coli*

The signal peptide region of the Enzyme B was replaced with that of maltose binding protein (malE) of *E. coli* as follows. Two oligonucleotides (SEQ ID No. 9 and 10) were synthesized with Applied Biosystem 381A DNA synthesizer and annealed to generate a double-stranded DNA fragment encoding a amino acid sequence (SEQ ID No. 11), MetLysIleLysThrGlyAlaArgIleLeuAlaLeuSerAlaLeuThrThrMetMetPheSer AlaSerAlaLeuAla(Gln), which was, then, treated with T4 polynucleotide kinase [J. Biol. Chem., 259, 10606-10613, (1984)]. pSSB203 (see Example 3) was digested with the restriction enzyme *Sph*I, treated with T4 DNA polymerase and digested with *Bst*P1. The resulting 1.72 kb DNA fragment carrying Enzyme B gene without the region coding for the original signal sequence and the first amino acid residue (Gln) of the mature Enzyme B was isolated from an agarose gel after agarose gel electrophoresis. The *E. coli* expression vector, pTrc99A (Pharmacia Co., Uppsala, Sweden), which was digested with the restriction enzymes *Nco*I (at ATG start codon) and *Sma*I was ligated with above two DNA fragments. The resulting plasmid was designated as pTrcMal-EnzB and used to transform *E. coli* JM109. The transformant was grown in two 2-liter flasks each containing 600 ml of LB with 100 μg/ml ampicillin at 28°C and IPTG was added to 0.1 mM when cell concentration reached at about 1.5 OD600. Following the addition of IPTG, the cells were cultivated for an additional 3 - 4 hours. The cells were harvested by centrifugation (4,000 x g) at 25°C for 10 minutes, suspended with 500 ml of 30 mM Tris-HCl, pH 8.0, containing 20% sucrose at 25°C. After EDTA was added to 1 mM into the cell suspension, the cells were incubated with gentle shaking for 5 minutes at 25°C and collected by centrifugation (8,000 x g) at 4°C for 15 minutes. The cells were resuspended with 500 ml of ice cold 5 mM $MgSO_4$ solution and incubated with gentle shaking for 5 minutes at 4°C. The cell suspension was centrifuged at 8,000 x g for 10 minutes at 4°C to obtain a supernatant as a cold osmotic shock extract, which was found to contain the Enzyme B protein (a molecular weight of 60,000) with the purity more than 50 - 60% by SDS-PAGE analysis. The supernatant was first supplemented with Tris-HCl, pH 8.0, to 20 mM, and incubated at 25°C firstly with EDTA at 10 mM final concentration for 10 min, secondly with $CaCl_2$ at 20 mM final concentration for 10 minutes and lastly with PQQ at 25 μM final concentration. For stabilization of the enzyme, α-methyl-D-glucoside (a competitive inhibitor) was added to 20 mM final concentration in the supernatant. The Enzyme B was completely purified by following two chromatographies. At first, the supernatant was loaded onto a Q-Sepharose column (1.6 x 12 cm) which had been equilibrated with 20 mM Tris-HCl, pH 8.0, containing 1 mM $CaCl_2$ and 20 mM α-methyl-D-glucoside, and the Enzyme B was eluted with 600 ml of 0 - 0.4 M NaCl linear gradient in the same buffer. A red protein peak eluted at about 0.25 M NaCl was collected and concentrated to about 0.5 ml by Centricon-30 (Amicon). Finally, the Enzyme B was passed through a SephacrylS-300HR column with 20 mM HEPES, pH 7.8, containing 0.2 M NaCl, 1 mM $CaCl_2$ and 20 mM α-methyl-D-glucoside. A red protein peak eluted around a molecular weight of 135,000 daltons position was collected as the final purified Enzyme B. Consequently, about 8 mg of the purified Enzyme B was obtained from 1.2 liters cultivation broth of *E. coli*.

Example 6. Host-vector system

A host-vector system for *G. oxydans* [DSM No. 4025] was established by using the conjugal mating system with a broad-host-range cosmid, pVK102. Initially, only one transconjugant was isolated from *G. oxydans* [DSM No. 4025] having nalidixic acid resistance. A new host, GOS2, was isolated from the transconjugants, *G. oxydans*[DSM No. 4025] carrying pVK102 by curing pVK102. A second host, GOS2R, was then derived from the GOS2 by adding rifampicin resistance (100 μg/ml), which enables easy selection of the transconjugants from the donor *E. coli.* The plasmid transfer frequency into GOS2R was $10^{-3} \sim 10^{-4}$ transconjugants/recipient. The 2KGA productivity of GOS2R, however, was about 10% lower than that of *G. oxydans* [DSM No. 4025]. The third host, GORS6-35, was obtained from *G. oxydans* [DSM No. 4025] by selecting the strain with rifampicin resistance, high 2KGA productivity and relatively high competence through a series of experiments, including the conjugation, curing and 2KGA fermentation.

(1) Isolation of GOS2

Resistance to nalidixic acid was added to *G. oxydans* [DSM No. 4025]. Cells of *G. oxydans* [DSM No. 4025] were streaked onto Trypticase Soy Broth (BBL, Becton Dickinson Microbiology Systems Cockeysville, MD. USA) (T) agar medium with 50 μg/ml of nalidixic acid (TN agar medium) and incubated at 27°C for 5 days. The resulting colonies were again streaked on the same agar plates to obtain a nalidixic acid-resistant *G. oxydans* DSM No. 4025, GON. The broad-host-range cosmid pVK102 ($Km^r$, $Tc^r$) was transferred from *E. coli* carrying pVK102 into the GON strain by the tri-parental conjugal mating as follows. A helper strain, *E. coli* carrying pRK2013 and a donor strain carrying pVK102 were cultivated in LB medium with 50 μg/ml of kanamycin at 37°C overnight. The cultures were transferred to fresh LB medium with kanamycin and incubated for 5-6 hours. Recipient strain, GON, was cultivated in TN liquid medium at 30°C overnight. *E. coli* and GON strains were separately centrifuged and re-suspended in equal- and one tenth- volumn of fresh T medium, respectively. One hundred μl of each cell suspension was mixed together and 30 μl portion of the mixture was spotted onto a nitrocellulose filter placed on the surface of a NS2 agar plate. Transconjugants were selected on the T agar medium containing 50 μg/ml of nalidixic acid and 50 μg/ml of kanamycin (TNK agar medium). Several colonies were obtained on the selection plates where many spontaneous mutants of *E. coli* ($Nal^r$, $Km^r$) colonies also appeared. The plasmid and chromosomal DNAs of the transconjugant candidates were prepared and compared with the authentic pVK102 and chromosomal DNA of *G. oxydans* DSM No. 4025 by restriction analysis and Southern-blot hybridization. Consequently, one transconjugant of *G. oxydans* [DSM No. 4025] carrying pVK102, GON8-1, was identified. The plasmid DNA prepared from GON8-1 was identical to that of pVK102 and replicable in *E. coli.* The chromosomal DNA of GON8-1 was identical to that of *G. oxydans* [DSM No. 4025].

To isolate strains that could work as hosts with higher competence for conjugal mating, the transconjugant GON8-1 was cured of the plasmid pVK102. GON8-1 was cultivated in T broth without antibiotics at 30°C for 2 days, 2% of the culture was transferred into fresh T broth. After three such cultivation cycles, the cells were spread on T agar plates, incubated at 27°C for 4 days, and the resulting colonies were picked onto TNK and TN agar plates to select $Km^S$ strains. One of the $Km^S$ strains was designated as GOS2 and was confirmed by Southern-blot hybridization not to be carrying any DNA region of pVK102. Then, pVK102 was transferred into strain GOS2 by a conjugal mating; this strain showed $10^2 \sim 10^3$ fold higher competence (namely $10^{-5} \sim 10^{-6}$ transconjugants / recipient) than *G. oxydans* [DSM No. 4025] did. (2) Isolation of GOS2R, a rifampicin-resistant mutant of GOS2.

Rifampicin resistant ($Rif^r$) mutants from GOS2 were isolated through repeated transfer of GOS2 cells onto T agar medium containing 20 - 100 μg/ml rifampicin; one of the $Rif^r$ strains was designated as GOS2R. Strain GOS2R showed very high competence; $10^{-2} \sim 10^{-3}$ and $10^{-4}$transconjugants /recipient on TRK agar (T agar medium containing 100 μg/ml rifampicin and 50 μg/ml kanamycin) plate and on TRT agar (T agar medium containing 100 μg/ml rifampicin and 3 μg/ml tetracycline ) plate, respectively.

2KGA productivity from L-sorbose by GOS2R was compared with that of *G. oxydans* [DSM No. 4025]. The cells maintained on NS2 agar medium were inoculated into 5 ml of the seed culture medium consisting of 8% L-sorbose (sterilized separtely), 0.05% glycerol, 0.25% $MgSO_4 \cdot 7H_2O$, 1.75% corn steep liquor, 5.0% baker's yeast, 1.5% $CaCO_3$, and 0.5% urea (sterilized separately) (pH 7.0 before sterilization) and incubated at 30°C for 24 hours. The resulting seed culture (5 ml) was inoculated into a 500-ml Erlenmeyer flask containing 50 ml of the production medium PMS10 consisting of 10% L-sorbose, (sterilized separtely), 0.05% glycerol, 0.25% $MgSO_4 \cdot 7H_2O$, 3% corn steep liquor, 6.25% baker's yeast, 1.5% $CaCO_3$, and 1.6% urea (sterilized separately) (pH 7.5 before sterilization) and incubated at 30°C for 4 days with shaking (180 rpm). The quantitative determination of 2KGA was assayed by high performance liquid chromatography. GOS2R and *G. oxydans* [DSM No. 4025] produced 87.3 and 97.3 g/l of 2KGA, respectively.

(3) Isolation of GORS6-35 as a host with high 2KGA productivity

To evaluate the self-cloning of AADH genes in the strain with the same productivity of 2KGA from L-sorbose as *G. oxydans* [DSM No. 4025], a new host was constructed by (i) adding rifampicin-resistance (200 μg/ml), (ii) introducing and curing pVK102, and (iii) selecting 2KGA high producer from L-sorbose. Thus obtained GORS6-35 shows the following two characteristics: (i) almost the same 2KGA productivity (about 100 g/l 2KGA from 10% L-sorbose) as the parent *G. oxydans* [DSM No. 4025]; and (ii) a competence ($10^{-6} \sim 10^{-7}$ transconjugants /recipient).

Example 7. Construction of promoter-replaced Enzyme B gene.

The promoter of Enzyme A gene (PA) is likely strong in *G. oxydans* [DSM No. 4025] because Enzyme A was found to be one of the highest-expressing proteins in amount in the cell when total cell free extract of *G. oxydans* [DSM No. 4025] was subjected to SDS-polyacrylamide gel electrophoresis and the resulting gel was stained with Coomassie Brilliant Blue R-250. The PA and another promoter, a promoter of kanamycin resistant gene of Tn5 (PTn5), which could express the kanamycin resistance in *G. oxydans* [DSM No. 4025], were attached to the structure gene with the SD sequence of Enzyme B gene as shown in Fig. 10.

Enzyme B gene-containing 2.3 kb *Hind*III fragment was inserted in M13 mp18 and the resulting phage DNA was subjected to site-directed mutagenesis carried out with T7-GEN™ In Vitro Mutagenesis Kit (TOYOBO Co., Ltd.,

Osaka Japan) according to the recommendations by the supplier (Fig. 9). To insert various promoters upstream of Enzyme B gene instead of Enzyme B promoter, *Bam*HI site was created upstream of the SD sequence. A primer for the mutagenesis, GTTAGCGCGGTGGATCCCCATTGGAGG (27-mer including *Bam*HI site, SEQ-IDNo. 12), were synthesized with Applied Biosystems 381A DNA synthesizer. The resulting *Bam*HI-*Hind*III fragment carries Enzyme B SD and structural genes without the Enzyme B promoter (PB).

Then promoter of Enzyme A gene (PA) was subcloned by PCR method using primers tagged with the sequences for the *Hind*III and *Bam*HI sites. The PCR reaction was carried out with GeneAmpTM DNA Amplification Reagent Kit (Takara Shuzo, Kyoto, Japan) with a thermal cycler, Zymoreactor II (Atto Corp., Tokyo, Japan). The reaction consists of pre-treatment before adding enzyme (94°C, 5 minutes.); 30 cycles of denaturation step (94°C, 1 minute.), annealing step (60°C, 1 minute.), and synthesis step (72°C, Iminute.); and post-treatment (72°C, 5 minutes.). Plasmid pSSA202 (pUC18-Enzyme A gene in 2.7kb *Hind*III) was used as the template DNA. The reaction mixture contained 200 μM of dNTPs, 1 μM of each primer, 1 ng of template DNA and 2.5 u of AmpliTaqTM DNA polymerase in the buffer supplied. Consequently, 300 bp fragment upstream from the SD sequence was amplified. The PCR product was inserted into pUC18 between *Hind*III and *Bam*HI sites and used for nucleotide sequencing; the amplified sequences do not have any mutations caused by misincorporation in PCR.

The promoter of the kanamycin resistant gene, PTn5, was first obtained as a *Hind*III-*Pst*I fragment from the plasmid pNeo (Pharmacia Co., Uppsala, Sweden). The *Hind*III-*Pst*I fragment was then inserted into the multicloning site of pUC18, and finally the PTn5 was excised as a *Hind*III-*Bam*HI fragment.

The *Hin*III-*Bam*HI fragments containing the PA and PTn5 promoters were inserted in the *Hind*III site of pUC18 together with *Bam*HI-*Hind*III fragment containing the PB promoter-removed Enzyme B structural gene. The *Hind*III fragments from the resulting plasmids were subcloned into pVK100 to produce pSSAP-B and pSSPTn5-B, which were transferred into GOS2R by conjugal mating as described in Example 6.

Example 8. 2KGA production by transconjugants of GOS2R in flask

(1) 2KGA production from D-sorbitol by GOS2R (pSSB103R) in single culture fermentation in flask.

The Enzyme B plasmid, pSSB103R, and the vector plasmid, pVK102, were introduced into GOS2R by a conjugal mating method as described in Example 6. The resulting transconjugants were maintained on NS2 agar medium containing 30 μg/ml tetracycline and subjected to 2KGA fermentation from D-sorbitol. The cells of the transconjugants were inoculated into 5 ml of the seed culture medium consisting of 8% D-sorbitol, 0.05% glycerol, 0.25% $MgSO_4 \cdot 7H_2O$, 1.75% corn steep liquor, 5.0% baker's yeast, 1.5% $CaCO_3$, and 0.5% urea (sterilized separately) (pH 7.0 before sterilization) and incubated at 30°C for 24 hours. The resulting seed culture (5 ml) was inoculated into a 500-ml Erlenmeyer flask containing 50 ml of three production media shown in Table 8 and incubated at 30°C for 3 days with shaking (180 rpm). As a result, GOS2R (pSSB103R) produced about 61.5, 71.5 and 73.0 g/l of 2KGA from 8%, 10% and 12% D-sorbitol, respectively, while GOS2R (pVK102) produced 19.5, 25.4 and 30.2 g/l 2KGA, respectively.

Table 8.

| | | (%) | |
|---|---|---|---|
| Ingredients | PMSL8 | PMSL10 | PMSL12 |
| D-Sorbitol | 8.0 | 10.0 | 12.0 |
| Glycerol | 0.05 | 0.05 | 0.05 |
| $MgSO_4 \cdot 7H_2O$ | 0.25 | 0.25 | 0.25 |
| CSL | 3.0 | 3.0 | 3.0 |
| Baker's yeast | 5.0 | 6.25 | 10 |
| Urea* | 1.25 | 1.6 | 2.0 |
| $CaCO_3$ | 1.5 | 1.5 | 1.5 |

pH 7.5 before sterilization
*: sterilized separately

(2) 2KGA production from D-sorbitol by GOS2R (pSSAP-B) and GOS2R (pSSPTn5-B) in single culture fermentation in flask.

The cells of GOS2R (pSSAP-B), GOS2R (pSSPTn5-B) and GOS2R (pSSB103R), GOS2R (pVK100) were cultivated in the PMSL10 production medium in Erlenmeyer flasks at 30°C for 3 days as described in Example 8 (1).

The amounts of 2KGA produced were shown in Table 9.

| Table 9. | The amount of 2KGA (g/l) | | |
|---|---|---|---|
| Strain | 1 day | 2 days | 3 days |
| GOS2R (pSSAP-B) | 47.2 | 67.0 | 67.7 |
| GOS2R (pSSPTn5-B) | 23.4 | 28.6 | 29.4 |
| GOS2R (pSSB103R) | 30.5 | 54.3 | 62.7 |
| GOS2R (pVK100) | 10.2 | 18.3 | 19.3 |
| GOS2R | 6.7 | 14.7 | 16.4 |

Example 9 2KGA production from D-sorbitol in 3-L Jar fermentations by single microorganism

(1) Single culture fermentation by GOS2R (pSSB103R)

Five ml portions of the seed culture prepared in test tubes as described in Example 8-(2) were transferred to four 500-ml Erlenmeyer flasks containing 50 ml of the same seed culture medium and incubated at 30°C for 24 hours with shaking (180 rpm). The resulting broth (200 ml of the seed culture) was inoculated into 3-L jar fermentor containing 1800 ml of the PMSL10 production medium containing 3ml of antifoam. The fermentor was operated at 30°C, 700 rpm and 0.5vvm. D-Sorbitol was fed in ways: (i) 200 ml of 50% D-sorbitol was fed in 6 hours from the $24^{th}$ to the $30^{th}$ hour; or (ii) 280 ml of 50% D-sorbitol was fed in 8.4 hours from the $24^{th}$ to the $32.3^{th}$ hour. As a result, 99.0 and 103.4 g/l 2KGA were produced by the fed-batch fermentations (i) and (ii), respectively in 51 hours.

Example 10. 2KGA production from D-sorbitol by Enzyme B gene-amplified GOS2R in mixed culture fermentation with *E. coli* in flask

(1) Mixed-culture fermentations with *B. megaterium, E. coli* and *P. putida*.

*B. megaterium* [DSM No. 4026], *E. coli* HB101 and *P. putida* [ATCC 21812], growth factor suppliers, were cultivated in 150 ml of the seed culture medium consisting of 0.3% yeast extract (Difco), 0.3% beef extract (Kyokuto Seiyaku, Tokyo, Japan), 3% corn steep liquor, 1% polypeptone (Kyokuto), 0.1% urea, 0.1% $KH_2PO_4$, 0.02% $MgSO_4{\cdot}7H_2O$, 2% L-sorbose, 0.1% $CaCO_3$ (pH 7.1 before sterilization) for 24 hours at 37, 37, and 30°C, respectively. Strain GOS2R (pSSB103R) was cultivated in two test tubes containing 5 ml of the seed culture medium as described in Example 8-(2) at 30°C for 24 hours. Four ml of GOS2R (pSSB103R) seed cultures and 3.5 ml of growth factor supplier seed culture were inoculated to a 500-ml of Erlenmeyer-flask containing 50 ml of the production medium for mixed culture fermentations consisting of 8% D-sorbitol, 0.01% $MgSO_4{\cdot}7H_2O$, 1% corn steep liquor, 0.1% $KH_2PO_4$, 0.6% $CaCO_3$, 1.5% urea

(sterilized separately) and antifoam (one drop per flask) (pH 7.0 before sterilization) and the flask was shaken at 30°C for 46.5 hours. As a result, mixed culture with B. *megaterium* DSM No. 4026, *E. coli* HB101 and *P. putida* ATCC 21812 produced 49.9, 54.1, 31.3 g/l 2KGA, respectively. (2) Mixed culture fermentation of GOS2R (pSSAP-B) with *E. coli* in flask.

Mixed culture fermentations by GOS2R (pSSAP-B) with *E. coli* was performed in the same manner as described above except for the seed culture medium for *E. coli* containing 2% D-sorbitol instead of 2% L-sorbose. From 10% of D-sorbitol, GOS2R (pSSAP-B) produced 73.7 g/l 2KGA in 48.5 hours.

Example 11. 2KGA production by recombinant AADH

The other reaction mixture containing 2.4 mg/ml each of purified Enzyme A and Enzyme B (purified according to Example 4), 50 mM Tris-HCl, pH7.5, 5 mM $CaCl_2$, 8 mg/ml BSA, 1mM PMS, 20 μg/ml PQQ, and 2% D-sorbitol was incubated at 30°C with gentle shaking for 20 hours. As a result, 0.25 g/l 2KGA (HPLC assay) and about 5 g/l L-sorbose (TLC assay) were produced.

Example 12. Production of aldehydes from alcohos, ketones from alcohols or carboxylic aicds and carboxylic acids from aldehydes.

Enzyme reactions with purified Enzyme B and various substrates were performed as described in Example 11. The resulting products were identified by TLC and/or HPLC as shown in Table 10.

Table 10

| Enzyme | Substrate | Product |
|---|---|---|
| Enzyme B | D-Glucose | D-Oluconic acid |
| | D-Sorbitol | L-Sorbose |
| | L-Sorbosone | 2KGA |
| | D-Mannitol | D-Fructose |
| | L-Idose | L-Idonic acid |
| | Glycerol | Dihydroxyacetone |
| | D-Gluconic acid | 5-Keto-D-gluconic acid |
| | D-Mannoic acid | S-Keto-D-mannoic acid |

Example 13. 2KGA and L-sorbose production by a transconjugant of *P. putida* (comparative Example)

A resting cell mixture (2 ml) containing 1% $CaCO_3$, 0.3% NaCl, 1mM PMS, 5 μg/ml PQQ, 2% L-sorbose and 10 OD600 unit-cells of nalidixic acid resistant ($Nal^r$) *P. putida* [ATCC 21812] carrying pSSA102R or pVK100 was incubated at 30°C with gentle shaking for 17 hours. As a result, $Nal^r$ *P. putida* [ATCC 21812] carrying pSSA102R or pVK100 produced 18.9 or 0.0 g/l of 2KGA, respectively.

A resting cell mixture (2 ml) containing 1% $CaCO_3$, 0.3% NaCl, 1mM PMS, 5 μg/ml PQQ, 2% D-sorbitol and 10 OD600 unit-cells of $Nal^r$ *P. putida* [ATCC 21812] with pSSB103R or with pVK100 was incubated at 30°C with gentle shaking for 17 hours. As a result, $Nal^r$ *P. putida* [ATCC 21812] carrying pSSB103R or with pVK100 produced 7.8 or 0.0 g/l of L-sorbose, respectively.

Example 14. Construction and characterization of chimera AADH enzymes (comparative Example)

(1) Construction of chimera AADH enzymes

To alternate substrate specificity of AADH enzymes, a variety of chimera enzymes between Enzymes A and B were constructed.

(i) Figure 2 shows the structure of the chimera genes by strategy I (restriction and ligation method). The restriction sites conserved in both genes, *Ava* I (nucleotide No. 603 of Enzyme A gene), *Eco*RI site (nucleotide No. 1084), and *Sal*I site (nucleotide No. 1470) (Fig. 7) were used for the construction. First, Enzyme A and B gene cassettes (2.7 kb and 2.3 kb Hind III fragments, respectively) were subcloned in the same direction in this order on pUC18 to produce the plasmid pSSAB201, and Enzyme B and A gene cassettes were also subcloned in the same direction in this order on pUC18 to produce pSSBA201 (Fig. 3). After partial digestion of these plasmids with each restriction enzyme, resulting digests were ligated and used to transform *E. coli* JM109. Ampicillin resistant transformants were analyzed for their plasmids, and Enzyme A gene-headed and Enzyme B-headed chimera gene cassettes with the expected *Hind*III fragment size of 2.7 kb and 2.3 kb, respectively, were selected. Thus constructed chimera gene cassettes were introduced into *Hind*III site of pVK102 to produce pSSA/B101R, pSSA/B102R, pSSA/B103R, pSSB/A101R, pSSB/A102R, and pSSB/A103R which encode Enzyme A/B1, EnzymeA/B2, EnzymeA/B3, EnzymeB/A1, EnzymeB/A2, and EnzymeB/A3, respectively, as shown in Fig. 2. These six plasmids were introduced into Nalr *P. putida* by a conjugal mating method as described in Example 1.

(ii) Figure 8 shows the scheme for constructing chimera genes by strategy II; in vivo homologous recombination method to construct chimeras recombinated at random positions for altering the substrate specificity of AADH enzymes. The principle of this method is as follows: (i) Locate two homologous genes to be recombinated tandem in one plasmid with selective marker; (ii) Cut it at restriction sites between the two genes, and transform *rec* $A^+$ *E. coli* cell with the linearized plasmid; (iii) Select transformants showing selective marker which carry circularized DNAs by recombination between the two genes at various positions. Two plasmids pSSAB201 and pSSBA201 which have Enzyme A and Enzyme B genes on pUC18 (Fig. 3) were linearized with pairs of restriction enzymes as shown in Fig. 8. *E. coli* JM101(*rec* $A^+$) was transformed with these linearized DNAs. Transformants were obtained at frequency of $10^1$-$10^2$ /μg DNA. To begin with, DNA size was determined to remove illegitimate recombinants. As a result, correct recombinants were obtained at ratio of 30%. *Xho*I-*Bal*I fragment in which Enzyme A gene lost about two-third of C-terminus was efficient to obtain chimeras recombinated within one-third of N-terminus. Next, the recombinants were classified into recombination site groups bordered by restriction sites of three *Sma*I, *Sph*I, *Sal*I and *Bal*I (Fig. 7). Thus constructed chimera genes were subcloned on pVK100 as *Hind*III cassette and the plasmids were introduced into $Nal^r$ *P. putida* by a conjugal mating method.

(2) Characterization of chimera AADH enzymes

(i) Characteristics of the chimeras obtained by restriction and ligation method

The chimera enzymes expressed in $Nal^r$ *P. putida* were characterized enzymatically by using soluble fractions of the cells of the transconjugants as described in Example 1. Eight substrates were used for the evaluation as shown in Fig. 11. Enzymes A/B 1 and A/B3 showed Enzyme A-type substrate specificity, although their expression level was lower than that of Enzyme A. On the other hand, Enzymes B/A1, B/A2, and B/A3 showed Enzyme B-type substrate specificity, although activity on n-propanol (Enzyme A type activity) became higher in accordans with the increase of the region from Enzyme A; the expression level of Enzyme B/A1 gene was about 2-fold higher than that of wild Enzyme B gene. As a result from the chimeras obtained by recombination and ligation method, it was concluded that N-terminal one third region of Enzyme A or Enzyme B determines its substrate specificity basically.

(ii) Characteristics of the chimeras obtained by homologous recombination method.

Among the chimeras obtained as above, seven out of eighteen chimera enzymes obtained from the chimera genes recombinated between *Sma*I2 and *Sal*I sites illustrated in Fig. 7 showed preferable substrate specificity. The seven chimera enzymes converted D-sorbitol to L-sorbose, not to D-glucose produced by Enzyme A, and converted L-sorbose to 2KGA like Enzyme A. The recombination sites were determined by nucleotide sequencing as described in Example 2. This type of chimeras having an approximate structure of " N-terminal 2/9 of Enzyme A + C-terminal 7/9 of Enzyme B " was classified as Enzyme superA-type. There were three Enzyme superA-type enzymes according to the recombinated site: Enzyme A/B21 (chimera consisting of Enzyme A part of amino acid residue No. 1 - 128 and Enzyme B part of No. 129 - 556), Enzyme A/B22 (chimera consisting of Enzyme A part of amino acid residue No. 1 - 125 and Enzyme B part of No. 126- 556) and Enzyme A/B25 (chimera consisting of Enzyme A part of amino acid residue No. 1 - 135 and Enzyme B part of No. 136 - 556). *P. putida* transconjugant expressing genes of Enzyme A/B21, Enzyme A/B22 or Enzyme A/B25 converted D-sorbitol to L-sorbose and did not convert D-sorbitol to D-glucose. The other type of chimera Enzyme A/B31 (Enzyme A part of amino acid residue No. 1 - 95 and Enzyme B part of No. 96 - 556) converted D-sorbitol to L-sorbose efficiently and did not convert L-sorbose to 2KGA; this chimera showed Enzyme B-type activity. Expression level of above mentioned chimeras was higher than that of wild Enzyme B because it was found that Enzyme B gene contains many rare codons but Enzyme A does not when the genes were analyzed with the program, Codon Preference (Wisconsin Sequence Analysis Package™, Genetics Computer Group).

(3) Improvement of codon usage in chimera genes

To further improve the chimeras, Enzyme A/B21, Enzyme A/B22, Enzyme A/B25 and Enzyme A/B31 in the view point of the preferable codon usage, the C-terminal two thirds consisting of Enzyme B residues were replaced with the C-terminal two thirds consisting of Enzyme A residues. Enzyme A/B21, Enzyme A/B22, Enzyme A/B25 and Enzyme A/B31 genes were used for constructing new chimera genes of Enzyme sA21 (Enzyme A part of amino acid residue No. 1 - 128, Enzyme B part of No. 129 - 180 and Enzyme A part of No. 180 - 556), Enzyme sA22 (Enzyme A part of amino acid residue No. 1 - 125, Enzyme B part of No. 126 - 180 and Enzyme A part of No. 180 - 556), Enzyme sA2 (Enzyme A part of amino acid residue No. 1 - 135, Enzyme B part of No. 136 - 180 and Enzyme A part of No. 180 - 556) and Enzyme sB (Enzyme A part of amino acid residue No. 1 - 95, Enzyme B part of No. 96 - 180 and Enzyme A part of No. 180 - 556) (Fig. 4). Actually, the replacement experiments for Enzyme sA2 and Enzyme sB were performed by partially digesting the plasmids, pUC18 carrying Enzyme sA gene and Enzyme B/A1 gene and pUC 18 carrying Enzyme A/B31 gene and Enzyme B/A1 gene with *Ava*I, ligating the resulting digests, transforming *E. coli* JM109, analyzing the plasmid structure of the transformants by restriction analysis, and determining the nucleotide sequence to confirm the expected recombination site, *Ava*I. The replacement experiments for Enzyme sA21 and Enzyme sA22 were performed by replacing the *Hind*III-*Ssp*I fragment of pSSsA2 encoding N-terminal part of Enzyme sA2 with the corresponding *Hind*III-*Ssp*I fragment containing recombinated site of Enzyme A/B21 or Enzyme A/B22 gene (Fig. 4).

(4) Kinetic properties of chimera enzymes

Tables 11 and 12 summarizes the kinetic properties of chimera enzymes, Enzyme sA2 and Enzyme sB in comparison with Enzyme A and Enzyme B, respectively.

Table 11. Enzyme sA2 vs Enzyme A

| | Enzyme sA2 | Enzyme A |
|---|---|---|
| $Km_{sorbose}$ | 128 mM | 36 mM |
| $Km_{sorbitol}$ | 2140 | 388 |
| $Km_{glucose}$ | 20 | - |

Products from L-sorbose in product assay with Enzyme sA2 and Enzyme A were 2KGA. Products from D-sorbitol in product assay with Enzyme sA2 and Enzyme A were L-sorbose with trace amount of D-glucose and D-glucose only, respectively. Thus, Enzyme sA2 showed desired characteristics for 2KGA production from D-sorbitol; L-sorbose/L-sorbosone dehydrogenase activity to produce 2KGA from L-sorbose like Enzyme A and D-sorbitol dehydrogenase activity to produce L-sorbose from D-sorbitol like Enzyme B.

Table 12. Enzyme sB vs Enzyme B

| | Enzyme sB | Enzyme B |
|---|---|---|
| $Km_{sorbitol}$ | 61 mM | 128 mM |
| $Ki_{sorbose}$ | 150 | 100 |

In comparison with Enzyme B, Enzyme sB showed higher affinity to D-sorbitol and lower affinity to L-sorbose which is the oxidation product of D-sorbitol and inhibitor in the conversion of D-sorbitol to L-sorbose.

Example 15. 2KGA production from D-sorbitol by GOS2R derivative strain amplified with chimera AADH enzymes (comparative Example)

For evaluating Enzyme sA2 and Enzyme sB, GOBΔK and GOI13 strains were constructed. GOBΔK was made from GOS2R by deleting the whole Enzyme B gene and instead inserting 1.28 kb $Km^r$ gene cassette isolated from pUC4K [4.1 kb, $Km^r$, $Amp^r$; Pharmacia, Uppsala, Sweden; Viera, J., and Messing, J., Gene 19:259, (1982)] by using a suicide vector pSUP201 [$Amp^r$, $Cm^r$, $mob^+$, a derivative of pBR325, Bio/Technology, 1:784-791, (1983)].

GOI13 was constructed from GOBΔK by replacing wild Enzyme A gene with Enzyme sB gene and deleting wild Enzyme A" gene replaced with gentamicin (Gm) resistant gene cassette with the suicide vector pSUP202 [$Amp^r$, $Cm^r$, $Tc^r$, $mob^+$, a derivative of pBR325, Bio/Technology, 1:784-791, {1983)]. The $Gm^r$ gene cassette was designed to have *Pst*I site at both ends by PCR amplification with the DNA fragment Tn5-GM [Sasagawa et al., Gene 56: 283-288, (1987)] as the template, and the resulting PCR product was inserted into *Pst*I site of pUC4K to produce pUC8G; $Gm^r$ gene can be isolated from pUC8G by digesting with *Eco*RI, *Bam*HI, *Sal*I, *or Pst*I.

(1) Effect of Enzyme sA2 amplification in 2KGA production

Plasmid pSSsA2, pVK100 with 2.7 kb *Hind*III cassette containing Enzyme sA2 gene, and its control plasmid pSSA102R, pVK102 with 2.7 kb *Hind*III cassette containing Enzyme A gene, were introduced into GOI13 by a conjugal mating method as described in Example 6. The resulting transconjugants were cultivated in PMSL10 medium at 30°C for 4 days as described in Example 8. GOI13 carrying pSSsA2 and pSSA102R produced 66.3 and 38.5 g/l of 2KGA, respectively, and 8.4 and 25.9 g/l of 2KD ( by-product of 2KGA produced from D-sorbitol via D-glucose and D-gluconate), respectively.

(2) Plasmids pSSsA21 and pSSsA22, which are pVK100 with 2.7 kb *Hind*III cassettes containing Enzyme sA21 and Enzyme sA22 genes, respectively (Fig. 4), were introduced into GOI13 by a conjugal mating method as described in Example 6. The resulting transconjugants were cultivated in PMSL10 medium at at 30°C for 4 days as described in Example 8. GOI13 carrying pSSsA21 and pSSsA22 produced 66.8 and 77.4 g/l of 2KGA, respectively, and 0.3 and 0.4 g/l of 2KD, respectively.

(3) Effect of Enzyme sB in 2KGA production

Plasmid pSSsB, pVK100 with 2.7 kb HindIII cassette containing Enzyme sB gene (Fig. 4) and its control plasmid pSSB103R, pVK102 containing 2.3 kb Enzyme B gene, were introduced into GOBΔK by a conjugal mating method. GOBΔK carrying pSSsB, GOBDK carrying pSSB103R, and GOBΔK were cultivated in PMSL8 medium as described in Example 8 (2) and produced 52.0, 46.8, and 1.1 g/l of 2KGA, respectively, and 6.9, 9.3, 32.3 g/l of 2KD, respectively.

GOI13, which carrys one copy of Enzyme sB on the chromosomal DNA without wild genes of Enzyme B, Enzyme A, and Enzyme A", was also cultivated in PMSL10 medium in 2 days. It produced 79.3 g/l of L-sorbose.

## Claims

1. A DNA molecule according to SEQ ID NO:4 or a DNA sequence which hybridizes under stringent hybridization and stringent washing conditions with said DNA molecule, said DNA encoding a polypeptide having alcohol and/or aldehyde dehydrogenase activity.

2. A recombinant enzyme preparation which comprises an enzymatic polypeptide selected from the group consisting of:

(a) an enzymatic polypeptide encoded by the DNA molecule according to claim 1 and
(b) an enzymatic polypeptide identified by SEQ ID NO:8 and having alcohol and/or aldehyde dehydrogenase activity.

3. A recombinant expression vector comprising one or more DNA molecules as defined by claim 1.

4. A recombinant host cell which carries the recombinant expression vector as claimed in claim 3 or one or more of DNA molecules defined by claim 1.

5. The recombinant host cell according to claim 4, wherein the host cell is *Gluconobacter oxydans* [DSM No. 4025].

6. A process for producing a recombinant enzyme preparation having an alcohol and/or aldehyde dehydrogenase activity as defined in claim 2, which comprises cultivating a recombinant host cell defined in any one of claims 4 to 5 in an appropriate culture medium and recovering said recombinant enzyme(s).

7. A process for producing an aldehyde, ketone or carboxylic acid product from alcohol or aldehyde which comprises converting alcohol or aldehyde into the product with the aid of a biochemical action of a recombinant host cell defined in claims 4 or 5.

8. A process for producing 2-keto-L-gulonic acid from L-sorbose and/or D-sorbitol which comprises converting L-sorbose and/or D-sorbitol into 2-keto-L-gulonic acid with the aid of a biochemical action of a recombinant host cell defined in claims 4 or 5.

9. A process for producing an aldehyde, ketone or carboxylic acid product from alcohol or aldehyde which comprises incubation of a reaction mixture containing a recombinant enzyme preparation defined in claim 2 and alcohol or aldehyde.

10. A process for producing 2-keto-L-gulonic acid which comprises incubation of a reaction mixture containing a recombinant enzyme preparation defined in claim 2 and L-sorbose and/or D-sorbitol.

11. A process for the production of L-ascorbic acid from 2-keto-L-gulonic acid characterized therein that a process as claimed in claim 8 or 10 is effected and the 2-keto-L-gulonic acid obtained by such process is transformed into L-ascorbic acid by a method known in the state of the art.

**Patentansprüche**

1. DNA-Molekül gemäß SEQ ID NO:4 oder DNA-Sequenz, die unter stringenten Hybridisierungs- und stringenten Maschbedinqunqen mit dem DNA-Molekül hybridisiert, wobei die DNA für ein Polypeptid mit Alkohol- und/oder Aidehyd-Dehydrogenase-Aktivität codiert.

2. Rekombinante Enzympräparation, die ein enzymatisches Polypeptid umfasst, das ausgewählt ist aus der Gruppe bestehend aus:

   (a) einem von dem DNA-Molekül gemäß Anspruch 1 codierten enzymatischen Polypeptid und
   (b) einem durch SEQ ID NO:8 identifizierten Polypeptid mit Alkohol- und/oder Aldehyd-Dehydrogenase-Aktivität.

3. Rekombinanter Expressionsvektor, umfassend ein oder mehrere DNA-Moleküle mit der durch Anspruch 1 gegebenen Bedeutung.

4. Rekombinante Wirtszelle, die den rekombinanten Expressionsvektor nach Anspruch 3 oder eines oder mehrere der DNA-Molekule mit der durch Anspruch 1 gegebenen Bedeutung trägt.

5. Rekombinante Wirtszelle gemäß Anspruch 4, bei der es sich um *Gluconobacter oxydans* (DSM Nr. 4025) handelt.

6. Verfahren zur Herstellung einer rekombinanten Enzympräparation mit einer Alkohol- und/oder Aldehyd-Dehydrogenase-Aktivität mit der in Anspruch 2 angegebenen Bedeutung, bei dem man eins rekombinante Wirtszelle mit der in einem der Ansprüche 4 bis 5 angegebenen Bedeutung in einem geeigneten Kulturmedium kultiviert und das rekombinante Enzym bzw. die rekombinanten Enzyme gewinnt.

7. Verfahren zur Herstellung eines Aldehyd-, Ketonoder Carbonsäureprodukts aus Alkohol oder Aldehyd, bei dem man Alkohol oder Aldehyd mit Hilfe einer biochemischen Maßnahme einer rekombinanten Wirtszelle mit der in Anspruch 4 oder 5 angegebenen Bedeutung in das Produkt umwandelt.

8. Verfahren zur Herstellung von 2-Keto-L-gulonsäure aus L-Sorbose und/oder D-Sorbit, bei dem man L-Sorbose und/oder D-Sorbit mit Hilfe einer biochemischen Maßnahme einer rekombinanten Wirtszelle mit der in Anspruch 4 oder 5 angegebene Bedeutung in 2-Keto-L-qulonsäure umwandelt.

9. Verfahren zur Herstellung eines Aldehyd-, Keton- oder Carbonsäureprodukts aus Alkohol oder Aldehyd, bei dem man ein eine rekombinante Enzympräparation mit der in Anspruch 2 angegebenen Bedeutung sowie Alkohol oder Aldehyd enthaltendes Reaktionsgemisch inkubiert.

10. Verfahren zur Herstellung von 2-Keto-L-gulonsäure, bei dem man ein eine rekombinante Enzympräparation mit der in Anspruch 2 angegebenen Bedeutung sowie L-Sorbose und/oder D-Sorbit enthaltendes Reaktionsgemisch inkubiert.

11. Verfahren zur Herstellung von L-Ascorbinsäure aus 2-Keto-L-gulonsäure, **dadurch gekennzeichnet, dass** ein Verfahren nach Anspruch 8 oder 10 ausgeführt und die mit einem solchen Verfahren erhaltene 2-Keto-L-gulonsäure mit einer im Stand der Technik bekannten Methode in L-Ascorbinsaure überführt wird.

## Revendications

1. Molécule d'ADN selon SEQ ID NO:4 ou une séquence d'ADN qui s'hybride dans des conditions stringentes d'hybridation et de lavage avec ladite molécule d'ADN, ledit an codant pour un polypeptide ayant l'activité de l'alcool et/ou de l'aldéhyde déshydrogénase.

2. Préparation d'enzyme recombinante qui comprend un polypeptide enzymatique choisi dans le groupe constitué :

   (a) d'un polypeptide enzymatique code par la molécule d'ADN selon la revendication 1 et
   (b) d'un polypeptide enzymatique identifié par SEQ ID NO:8 et ayant l'activité de l'alcool et/ou de l'aldéhyde déshydrogénase.

3. Vecteur d'expression recombinant comprenant une ou plusieurs molécules d'ADN telles que définies par la revendication 1.

4. Cellule hôte recombinante qui porte le vecteur d'expression recombinant selon la revendication 3 ou une ou plusieurs molécules d'ADN définies par la revendication 1.

5. Cellule hôte recombinante selon la revendication 4, dans laquelle la cellule hôte est *Gluconobacter oxydans* [DSM N° 4025].

6. Procédé de production d'une préparation d'enzyme recombinante ayant l'activité de l'alcool et/ou de l'aldéhyde déshydrogénase telle que définie dans la revendication 2, qui comprend la culture d'une cellule hôte recombinante définie dans l'une ou l'autre des revendications 4 et 5 dans un milieu de culture approprié et la a récupération de ladite ou desdites enzymes recombinantes.

7. Procédé de production d'un produit aldéhyde, cétone ou acide carboxylique à partir d'un alcool ou d'un aldéhyde qui comprend la transformation de l'alcool ou de l'aldéhyde en le produit à l'aide d'une action biochimique d'une cellule hôte recombinante définie dans la revendication 4 ou 5.

8. Procédé de production d'acide 2-céto-n-gulonique à partir de L-sorbose et/ou de D-sorbitol qui comprend la transformation du L-sorbose et/ou du D-sorbitol en acide 2-céto-L-gulonique à l'aide d'une action biochimique d'une cellule hôte recombinante définie dans la revendication 4 ou 5.

9. Procédé de production d'un produit aldéhyde, cétone ou acide carboxylique à partir d'un alcool ou d'un aldéhyde qui comprend l'incubation d'un mélange réactionnel contenant une préparation d'enzyme recombinante définie dans la revendication 2 et de l'alcool ou de l'aldéhyde.

**10.** Procédé de production d'acide 2-céto-L-gulonique qui comprend l'incubation d'un mélange réactionnel contenant une préparation d'enzyme recombinante définie dans la revendication 2 et du L-sorbose et/ou du D-sorbitol.

**11.** Procédé de production d'acide L-ascorbique à partir d'acide 2-céto-L-gulonique, **caractérisé en ce qu'**un procédé selon la revendication 8 ou 10 est effectué et l'acide 2-céto-L-gulonique obtenu par un tel procédé est transformé en acide L-ascorbique par une méthode connue dans l'état de la technique.

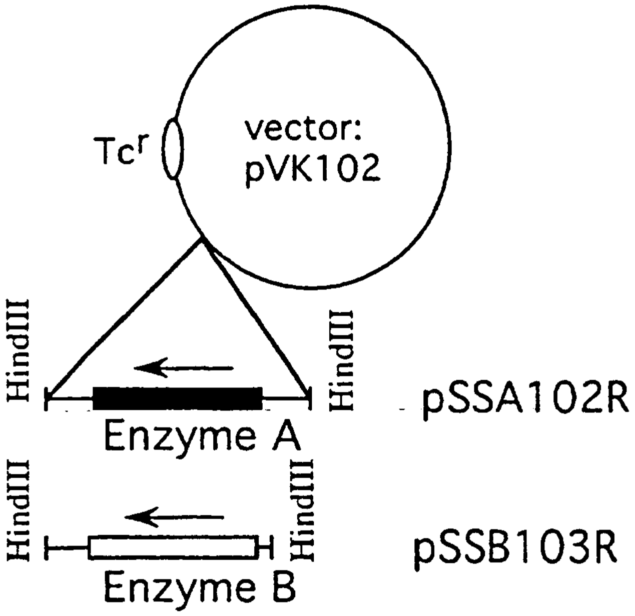
vector:
pVK102
Tc^r
HindIII
HindIII
pSSA102R
Enzyme A
HindIII
HindIII
pSSB103R
Enzyme B

Fig. 1.

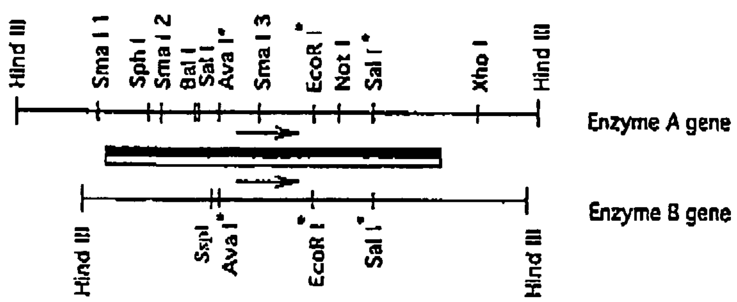


*: AvaI, EcoRI, SalI sites used for constructing chimera genes shown in Figs. 2 and 6.

Fig. 7.

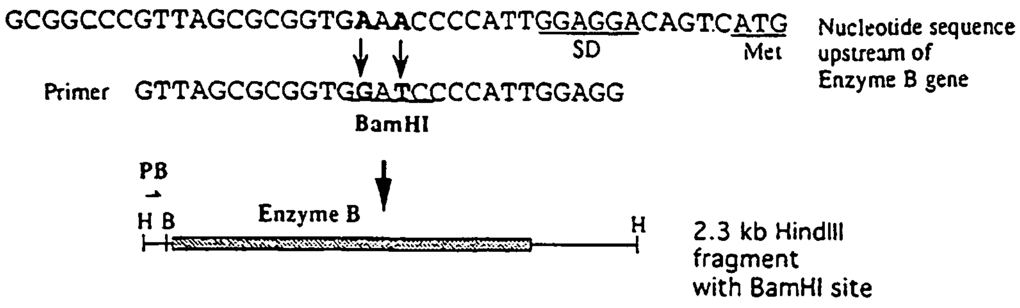
GCGGCCCGTTAGCGCGGTGAAACCCCATTGGAGGACAGTCATG
SD
Met
Nucleotide sequence upstream of Enzyme B gene
Primer GTTAGCGCGGTGGATCCCCATTGGAGG
BamHI
PB
H B
Enzyme B
H
2.3 kb HindIII fragment with BamHI site

Fig. 9.

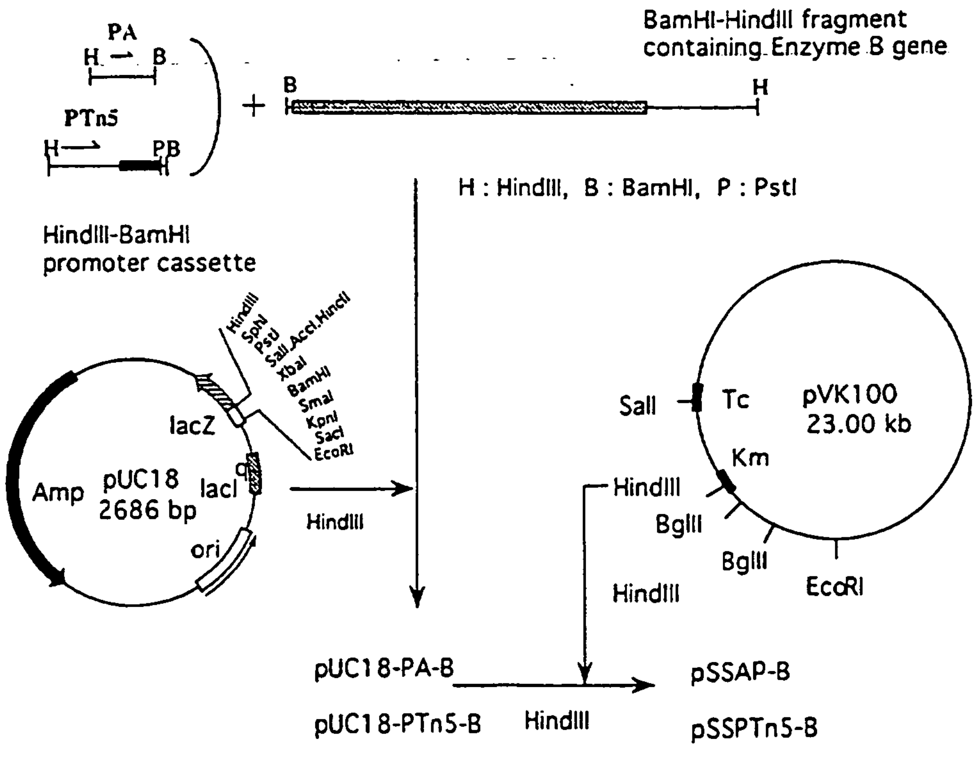
PA
H B
PTn5
H PB
BamHI-HindIII fragment containing Enzyme B gene
B
H
H : HindIII, B : BamHI, P : PstI
HindIII-BamHI promoter cassette
HindIII
SphI
PstI
SalI,AccI,HincII
XbaI
BamHI
SmaI
KpnI
SacI
EcoRI
lacZ
Amp
pUC18
2686 bp
lacI
ori
HindIII
SalI
Tc
pVK100
23.00 kb
Km
HindIII
BglII
BglII
HindIII
EcoRI
pUC18-PA-B
pUC18-PTn5-B
HindIII
pSSAP-B
pSSPTn5-B

Fig. 10.

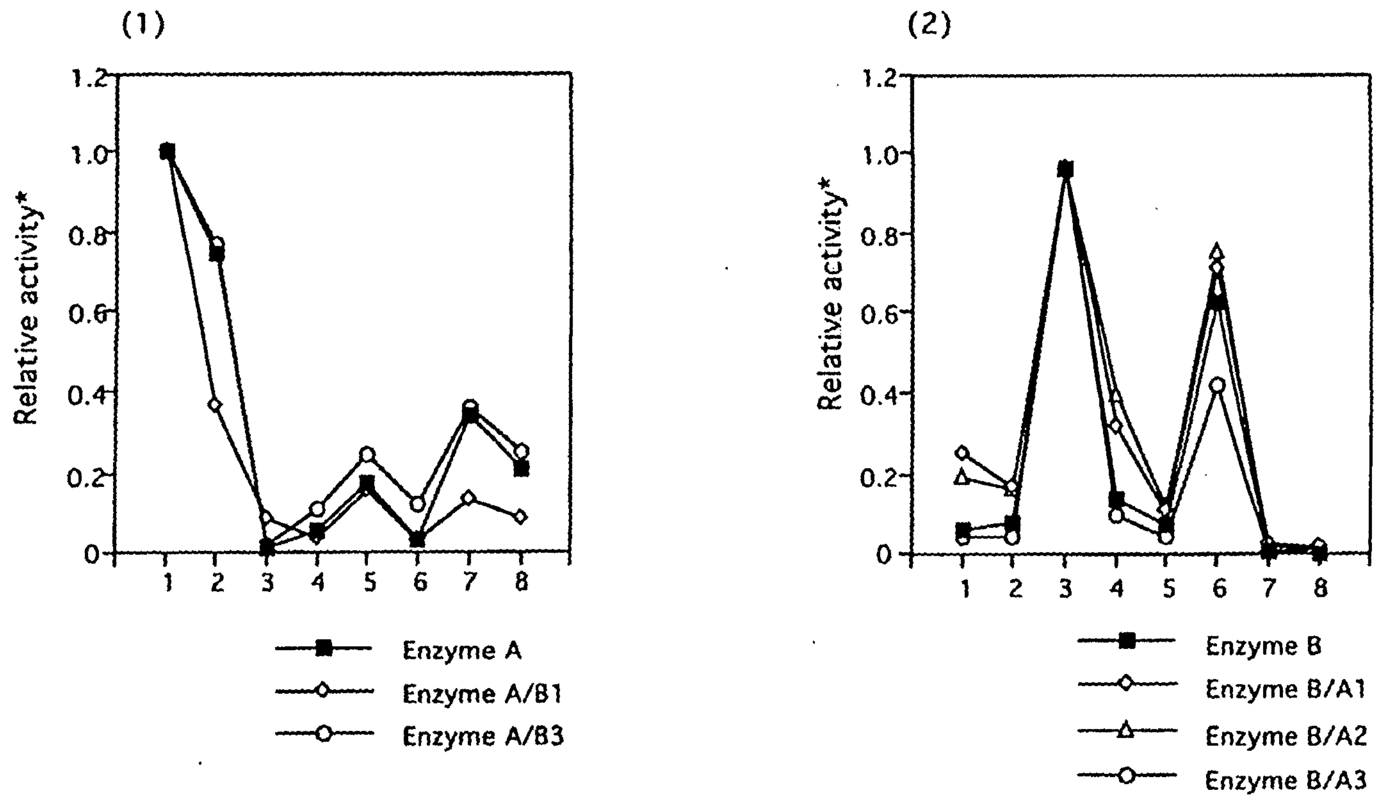


1. n-Propanol, 2. Isopropanol, 3. D-Glucose, 4. L-Sorbosone
5. D-Sorbitol, 6. D-Mannitol, 7. L-Sorbose, 8. D-Fructose

*Enzyme activity was normalized relative to activity for n-propanol (1), or D-glucose (2). Enzyme A/B2 was excepted because of its low expression in *P. putida.*

Fig. 11.

```
Enzyme A   1 : QVTPVTDELL ANPPAGEWIS YGQNQENYRH SPLTQITTEN VGQLQLVWAR GMQPGKVQVT
               **** ***** *********  ** ******* *******  * ********** **  * ****
Enzyme B   1 : QVTPITDELL ANPPAGEWIN YGRNQENYRH SPLTQITADN VGQLQLVWAR GMEAGAVQVT

          61 : PLIHDGVMYL ANPGDVIQAI DAKTGDLIWE HRRQLPNIAT LNSFGEPTRG MALYGTNVYF
               * ******** *********  ** ******* ******  ** **  *   **  *****  **
          61 : PMIHDGVMYL ANPGDVIQAL DAQTGDLIWE HRRQLPAVAT LNAQGDRKRG VALYGTSLYF

                                                                             *
                                                                          AvaI
         121 : VSWDNHLVAL DTATGQVTFD VDRGQGED-M VSNSSGPIVA NGVIVAGSTC QYSPFGCFVS
                ****** ** *  **** ** * ** ***    **  ***** ********** **** *** *
         121 : SSWDNHLIAL DMETGQVVFD VERGSGEDGL TSNTTGPIVA NGVIVAGSTC QYSPYGCFIS

         180 : GHDSATGEEL WRNYFIPRAG EEGDETWGND YEARWMTGAW GQITYDPVTN LVHYGSTAVG
               ********** *** ***  * **********  ******* * ********** ** **** **
         181 : GHDSATGEEL WRNHFIPQPG EEGDETWGND FEARWMTGVW GQITYDPVTN LVFYGSTGVG

         240 : PASETQRGTP GGTLYGTNTR FAVRPDTGEI VWRHQTLPRD NWDQECTFEM MVTNVDVQPS
               ********** ********** ********** ********** ********** ** *******
         241 : PASETQRGTP GGTLYGTNTR FAVRPDTGEI VWRHQTLPRD NWDQECTFEM MVANVDVQPS

                                                        *
                                                    EcoRI
         300 : TEMEGLQSIN PNAATGERRV LTGVPCKTGT MWQFDAETGE FLWARDTNYQ NMIESIDENG
                *****  ** ********** *** ****** ** ***  ** *********  *** **** *
         301 : AEMEGLRAIN PNAATGERRV LTGAPCKTGT MWSFDAASGE FLWARDTNYT NMIASIDETG

         360 : IVTVNEDAIL KELDVEYDVC PTFLGGRDWP SAALNPDSGI YFIPLNNVCY DMMAVDQEFT
                ******* * ********** *********  ******* ** ** **** ** * *******
         361 : LVTVNEDAVL KELDVEYDVC PTFLGGRDWS SAALNPDTGI YFLPLNNACY DIMAVDQEFS

                                                             *
                                                         SalI
         420 : SMDVYNTSNV TKLPPGKDMI GRIDAIDIST GRTLWSVERA AANYSPVLST GGGVLFNGGT
                 ******     ** **     ********** ****** **  **********  *** *****
         421 : ALDVYNTSAT AKLAPGFENM GRIDAIDIST GRTLWSAERP AANYSPVLST AGGVVFNGGT

         480 : DRYFRALSQE TGETLWQTRL ATVASGQAIS YEVDGMQYVA IAGGGVSYGS GLNSALAGER
               ********** ******* ** **** ***** ** ** ** * *  **  **   **  ** *
         481 : DRYFRALSQE TGETLWQARL ATVATGQAIS YELDGVQYIA IGAGGLTYGT QLNAPLA-EA

         540 : VDSTAIGNAV YVFALPQ
                ***  ***  *******
         540 : IDSTSVGNAI YVFALPQ
```

* : Nucleotide sequences encoding these regions are the restriction sites for AvaI, EcoRI, and SalI which were used for constructing chimera genes shown in Fig. 2.

Fig. 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 51040154 A
- EP 0221707 A
- EP 0278447 A
- EP 88116156 A
- EP 747483 A
- EP 0278477 A
- WO 8906688 A
- WO 97115801 A


### Non-patent literature cited in the description


- *Acta Microbiologica Sinica,* 1981, vol. 21 (2), 185-191
- **Sambrook et al.** Molecular Cloning. Cold Spring Harbour Laboratory Press, 1989
- **Marmur J.** *J. Mol. Biol.,* 1961, vol. 3, 208
- *Methods in Enzymology,* 1981, vol. 73, 46
- **Sanger F. et al.** *Proc. Natl. Acad. Sci.USA,* 1977, vol. 74, 5463-5467
- **Berg, D. E. ; C. M. Berg.** *Bio/Technology,* 1983, vol. 1, 417-435
- *J. Bacteriol.,* 1978, vol. 134, 1141-1156
- **Maniatis et al.** Molecular cloning: a laboratory manual. Cold Spring Harbor Laboratory, 1982
- **Murray, N. E. ; W. J. Brammar ; K. Murray.** *Mol. Gen. Genet.,* 1977, vol. 150, 53-61
- **Tra.** *Bio/Technology,* 1983, vol. 1, 784-791
- **T. Inoue et al.** *J. Bacteriol.,* vol. 171, 3115-3122
- **T. Tamaki et al.** *B. B. A.,* vol. 1088, 292-300
- **N. Harms et al.** *J. Bacteriol.,* vol. 169, 3966-3975
- **S. M. Machlin et al.** *J. Bacteriol.,* vol. 170, 4739-4747
- **D. J. Anderson et al.** *Gene,* vol. 90, 171-176
- **Edman.** *Acta Chem. Scand.,* 1950, vol. 4, 283-293
- *J. Biol. Chem.,* 1984, vol. 259, 10606-10613
- **Viera, J. ; Messing, J.** *Gene,* 1982, vol. 19, 259
- *Bio/Technology,* 1983, vol. 1, 784-791
- **Sasagawa et al.** *Gene,* 1987, vol. 56, 283-288